# EUROPEAN PATENT SPECIFICATION

(11) **EP 4 320 584 B1**
(45) Date of publication and mention of the grant of the patent: **25.11.2026**
(21) Application number: 22881212.9
(22) Date of filing: 05.09.2022
(51) Int. Cl.: G06Q 50/10, G08B 21/02, G16Y 10/60, G16Y 20/10, G16Y 40/10, G16Y 40/50, G01S 13/00, G06N 20/00

(54) **METHOD, AND DEVICE FOR PROVIDING HUMAN WELLNESS RECOMMENDATION BASED ON UWB BASED HUMAN ACTIVITY DETECTION**
VERFAHREN UND VORRICHTUNG ZUR BEREITSTELLUNG VON EMPFEHLUNGEN FÜR MENSCHLICHE WOHLBEFINDEN AUF BASIS VON UWB-BASIERTER ERKENNUNG MENSCHLICHER AKTIVITÄTEN
PROCÉDÉ ET DISPOSITIF POUR FOURNIR UNE RECOMMANDATION EN MATIÈRE DE BIEN-ÊTRE HUMAIN SUR LA BASE D'UNE DÉTECTION D'ACTIVITÉ HUMAINE BASÉE SUR UNE BANDE ULTRA-LARGE

(30) Priority: 14.10.2021 IN 202141046903
(43) Date of publication of application: 14.02.2024
(73) Proprietor: Samsung Electronics Co., Ltd., Suwon-si, Gyeonggi-do 16677 (KR)
(72) Inventor: MOULIK, Sreedeep, Suwon-shi, Gyeonggi-do, 16677 (KR); BISWAS, Raunaq, Suwon-si, Gyeonggi-do, 16677 (KR); AGRAWAL, Rahul, Suwon-si, Gyeonggi-do, 16677 (KR); CHAKRABORTY, Anuran, Suwon-si, Gyeonggi-do, 16677 (KR)
(74) Representative: Gulde & Partner
(86) International application number: PCT/KR2022/013281
(87) International publication number: WO 2023/063582

(56) References cited:
- EP-A1- 3 695 783
- WO-A1-2019/199580
- KR-A- 20210 001 170
- US-A1- 2016 379 475
- US-A1- 2018 025 126
- US-A1- 2021 049 890
- US-A1- 2021 110 692
- US-A1- 2021 173 045

## Description

### [Technical Field]

The disclosure relates to the field of Internet of Things (IoT). More particularly, the disclosure relates to a method and a human wellness recommendation system for providing human wellness recommendation using Ultra-Wideband (UWB) based human activity detection.

### [Background Art]

In an loT environment, cameras or/and wearable devices are typically used to identify a person. Furthermore, the information gathered from the cameras or/and the wearable devices may be used to identify activity (or movement) of the person. However, use of cameras and/or wearable devices have their limitations. For example, facial recognition of a person may fail due to poor camera frame capture quality and limited view angle of a camera. Additionally, view angle of the camera can't penetrate obstruction such as a furniture or a wall, which prevents identifying an activity of the person on the other side of obstruction. The models that use information captured by cameras can't detect speed and continuous activity (or movement) pattern of the person. Also, use of a wearable device to alert the person may not always work as the wearable device may be kept for charging or the person may not be wearing the wearable device when an alert is sent to the person. Hence, there is a need to provide wellness risk alert and/or a wellness solution to a user/users based on detection of user activity that overcomes above drawbacks. EP3695783A1 relates to a wireless gait recognition method.

The above information is presented as background information only to assist with an understanding of the disclosure. No determination has been made, and no assertion is made, as to whether any of the above might be applicable as prior art with regard to the disclosure.

### [Disclosure]

### [Technical Solution]

The present invention is defined by the appended set of claims. According to an embodiment, a method performed by an electronic device for providing human wellness recommendations based on Ultra-Wideband (UWB) based human activity detection is provided as defined by the appended set of claims.

According to an embodiment, an electronic device for providing human wellness recommendations based on Ultra-Wideband (UWB) based human activity detection is provided as defined by the appended set of claims.

According to an embodiment, a computer-readable storage medium, having a computer program stored thereon that performs, when executed by a processor, is provided as defined by the appended set of claims.

### [Description of Drawings]

The above and other aspects, features, and advantages of certain embodiments of the disclosure will be more apparent from the following description taken in conjunction with the accompanying drawings, in which:
FIG. 1A illustrates an environment for providing human wellness recommendation using Ultra-Wideband (UWB) based human activity detection according to an embodiment of the disclosure;
FIG. 1B illustrates an example for providing human wellness recommendation using Ultra-Wideband (UWB) based human activity detection according to an embodiment of the disclosure;
FIG. 2A shows a detailed block diagram of a human wellness recommendation system according to an embodiment of the disclosure;
FIGS. 2B and 2C illustrates working of a profile and activity identifier module of the human wellness recommendation system according to various embodiments of the disclosure;
FIG. 2D illustrates working of a device and event correlation module of the human wellness recommendation system according to an embodiment of the disclosure;
FIG. 2E illustrates working of a wellness analyzer module of the human wellness recommendation system according to an embodiment of the disclosure;
FIG. 2F illustrates working of an alert generator module of the human wellness recommendation system according to an embodiment of the disclosure;
FIG. 2G illustrates working of an action optimizer module of the human wellness recommendation system according to an embodiment of the disclosure;
FIG. 3 shows a flowchart illustrating a method for providing human wellness recommendation using UWB based human activity detection according to an embodiment of the disclosure;
FIGS. 4A and 4B illustrate first example for providing human wellness recommendation using UWB based human activity detection according to various embodiments of the disclosure;
FIGS. 5A and 5B illustrate second example for providing human wellness recommendation using UWB based human activity detection according to various embodiments of the disclosure;
FIGS. 6A and 6B illustrate third example for providing human wellness recommendation using UWB based human activity detection according to various embodiments of the disclosure;
FIGS. 7A and 7B illustrate fourth example for providing human wellness recommendation using UWB based human activity detection according to various embodiments of the disclosure; and
FIGS. 8A and 8B illustrate fifth example for providing human wellness recommendation using UWB based human activity detection according to various embodiments of the disclosure.

The same reference numerals are used to represent the same elements throughout the drawings.

Similarly, it will be appreciated that any flowcharts, flow diagrams, state transition diagrams, pseudo code, and the like represent various processes which may be substantially represented in computer readable medium and executed by a computer or processor, whether or not such computer or processor is explicitly shown.

### [Mode for Invention]

The following description with reference to the accompanying drawings is provided to assist in a comprehensive understanding of various embodiments of the disclosure as defined by the claims. It includes various specific details to assist in that understanding, but these are to be regarded as merely exemplary. Accordingly, those of ordinary skill in the art will recognize that various changes and modifications of the various embodiments described herein can be made without departing from the scope of the disclosure. In addition, descriptions of well-known functions and constructions may be omitted for clarity and conciseness.

The terms and words used in the following description and claims are not limited to the bibliographical meanings, but are merely used by the inventor to enable a clear and consistent understanding of the disclosure. Accordingly, it should be apparent to those skilled in the art that the following description of various embodiments of the disclosure is provided for illustration purposes only and not for the purpose of limiting the disclosure as defined by the appended claims.

It is to be understood that the singular forms "a," "an," and "the" include plural referents unless the context clearly dictates otherwise. Thus, for example, reference to "a component surface" includes reference to one or more of such surfaces.

In the disclosure, the word "exemplary" is used herein to mean "serving as an example, instance, or illustration." Any embodiment or implementation of the disclosure described herein as "exemplary" is not necessarily to be construed as preferred or advantageous over other embodiments.

While the disclosure is susceptible to various modifications and alternative forms, specific embodiment thereof has been shown by way of example in the drawings and will be described in detail below. It should be understood, however that it is not intended to limit the disclosure to the particular forms disclosed, but on the contrary, the disclosure is to cover all modifications, equivalents, and alternatives falling within the scope of the disclosure.

The terms "comprises", "comprising", or any other variations thereof, are intended to cover a non-exclusive inclusion, such that a setup, device or method that comprises a list of components or steps does not include only those components or steps but may include other components or steps not expressly listed or inherent to such setup or device or method. In other words, one or more elements in a system or apparatus proceeded by "comprises 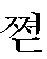 a" does not, without more constraints, preclude the existence of other elements or additional elements in the system or method.

In the following detailed description of the embodiments of the disclosure, reference is made to the accompanying drawings that form a part hereof, and in which are shown by way of illustration specific embodiments in which the disclosure may be practiced. These embodiments are described in sufficient detail to enable those skilled in the art to practice the disclosure, and it is to be understood that other embodiments may be utilized and that changes may be made without departing from the scope of the disclosure. The following description is, therefore, not to be taken in a limiting sense.

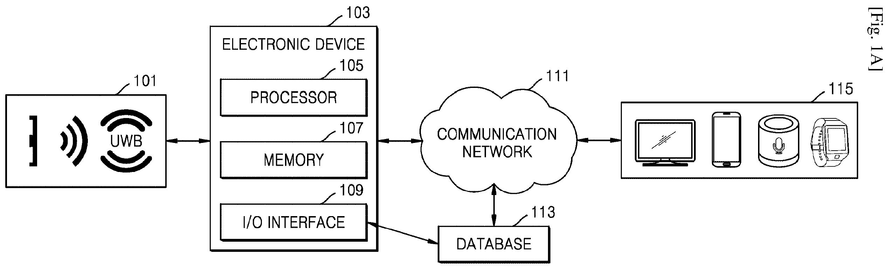

FIG. 1A illustrates an environment for providing human wellness recommendation based on Ultra-Wideband (UWB) based human activity detection according to an embodiment of the disclosure.

Referring to FIG. 1A, the environment includes UWB based sensors 101, an electronic device (alternatively referred as a human wellness recommendation system) 103, a communication network 111, a database 113, and at least one IoT enabled device 115. The UWB based sensors 101 refers to one or more UWB based sensors 101. The UWB based sensors 101 are placed or installed in an IoT environment. The loT environment may refer to a house, an office space, or any enclosed space in which one or more UWB based sensors 101 are installed. For example, a house may have a plurality of rooms (also referred as locations), such as a hall, a dining room, a bedroom, a kitchen and the like. Each of the plurality of rooms is installed with one or more UWB based sensors 101. Analogously, an office space or any enclosed space may have a plurality of rooms with each of the plurality of rooms installed with one or more UWB based sensors 101. The UWB based sensors 101 collect multiple angular data in a time-series manner using omni-directional antenna. The time-series manner refers to multiple angular data taken at successive equally spaced points in time. The UWB based sensors 101 transmit signal (Tx) to stationary and non-stationary objects present in a room (also, referred as location) in which the UWB based sensors 101 are installed. In return, the UWB based sensors 101 receive scattered signals (Rx) reflected from the stationary and non-stationary objects present in the room (location). The signals transmitted are in a pulse (i.e., non-continuous) form. The scattered signals reflected from the stationary and non-stationary objects present in the room (location) comprise of properties such as Signal Strength (SS), Time of Delayed Arrival (TDA), Time of Arrival (ToA) and Angle of Arrival (AoA). The properties of the scattered signals such as SS, TDA, ToA and AoA are, also referred as multiple angular data. Thereafter, the UWB based sensors 101 send the collected multiple angular data to the human wellness recommendation system 103. The UWB based sensors 101 are connected to the electronic device (human wellness recommendation system) 103 in a wireless manner or in a wired manner.

The electronic device (the human wellness recommendation system) 103 receives the multiple angular data from the UWB based sensors 101. The human wellness recommendation system 103 includes a processor 105, a memory 107 and an Input/Output (I/O) interface 109. The I/O interface 109 is configured to receive the multiple angular data from the UWB based sensors 101 as an input and provide at least one of a wellness risk alert and/or a wellness solution (to be described later) using the at least one IoT enabled device 115 in the loT environment as an output. The I/O interface 109 communicate with the at least one loT enabled device 115 using the communication network 111 that may employ communication protocols/methods such as, without limitation, Bluetooth, cellular e.g., Code-Division Multiple Access (CDMA), High-Speed Packet Access (HSPA+), Global System for Mobile communications (GSM), Long-Term Evolution (LTE), Worldwide interoperability for Microwave access (WiMax), or the like.

The multiple angular data received from the UWB based sensors 101 by the I/O interface 109 is stored in the memory 107. The memory 107 is communicatively coupled to the processor 105 of the human wellness recommendation system 103. The memory 107 also stores instructions which cause the processor 105 to execute the instructions for providing human wellness recommendation using UWB based human activity detection. The memory 107 may include memory drives, removable disc drives, and the like. The memory drives may further include a drum, magnetic disc drive, magneto-optical drive, optical drive, Redundant Array of Independent Discs (RAID), solid-state memory devices, solid-state drives, and the like.

The processor 105 may include at least one data processor for providing human wellness recommendation using UWB based human activity detection. The processor 105 may include specialized processing units such as integrated system (bus) controllers, memory management control units, floating point units, graphics processing units, digital signal processing units, and the like.

The human wellness recommendation system 103 may exchange data with the database 113 directly or through the communication network 111 that may employ communication protocols/methods such as Bluetooth, cellular e.g., Code-Division Multiple Access (CDMA), High-Speed Packet Access (HSPA+), Global System for Mobile communications (GSM), Long-Term Evolution (LTE), Worldwide interoperability for Microwave access (WiMax), or the like to. The database 113 stores historic physical profile and historic activity profile of users. The database 113 is initially populated or stored with the historic physical profile and the historic activity profile of users during training phase (to be described later). The historic physical profile comprises information associated with user which may include, but is not limited to, height, user body type, user shape, user age, user gender and stage type, user movement, user average speed of movement, and restricted user movement. The user body type may include, but is not limited to, lean body type, fat body type, muscular body type and the like. The user shape comprises of, but not limited to, round shape, straight shape, bend (or slouch) shape and the like. The user gender and stage type may include, but is not limited to, kid, teen, youth, adult, male, female, and the like. The user movement may include, but is not limited to, left torso movement, right torso movement, right hand movement, left hand movement and the like. The user average speed of movement refers to location specific speed of body movement of a user. The restricted user movement refers to less frequently performed body movement by a particular user. The historic activity profile of users comprises of any kind of physical activity such as, but not limited to, walking, moving, running, crawling, sitting, standing, jumping, bending, cleaning, eating and the like. The database 113 is hosted on a cloud server or on an edge server.

The historic physical profile and the historic activity profile of users in the database 113 is updated by the human wellness recommendation system 103 or by the user or by both at any point in time.

The human wellness recommendation system 103 may provide at least one of a wellness risk alert and/or a wellness solution (to be described later) using the at least one IoT enabled device 115 in the IoT environment. The at least one IoT enabled device 115 may include, but is not limited to, electronic appliances, electronic devices or any object embedded with electronics, sensors and Internet connectivity. For example, the at least one IoT enabled device 115 may be a mobile terminal, a speaker, a smartwatch, a light bulb, or the like. A person skilled in the art would understand that any loT devices, not mentioned explicitly, may also be used as the loT enabled device 115 in the disclosure. The human wellness recommendation system 103 is communicatively connected to the at least one IoT enabled device 115 via the communication network 111.

Hereinafter, the operation of the electronic device (the human wellness recommendation system) 103 is explained in two parts: (1) the first part is a training phase of the human wellness recommendation system 103, and (2) the second part is an application phase or a prediction phase of the human wellness recommendation system 103 for providing human wellness recommendation using UWB based human activity detection.

During the training phase, the UWB based sensors 101 collect and obtain multiple angular data in a time-series manner. The multiple angular data may be collected/taken at successive equally spaced points in time, for example, every 2 min. Also, during the training phase, the multiple angular data is collected over a period of days, such as at least 7 days. The multiple angular data comprising properties of scattered signals such as SS, TDA, ToA and AoA is sent to the human wellness recommendation system 103 in a wireless manner or in a wired manner. Using SS, TDA, ToA and AoA and propagation geometry (also referred as arrangement) of the UWB based sensors 101 installed in a room (location) in the loT environment, the human wellness recommendation system 103 records position, size, direction, movement of each stationary and non-stationary objects in a room (location) in the loT environment. The stationary objects include objects that are usually stationary in a room (location) such as a table, chairs, furniture, a desk, a television, a fridge, a washing machine, or the like. The non-stationary objects include objects that are usually moving in a room (location) such as human beings (user or users). The human wellness recommendation system 103 monitors the multiple angular data in a room (location) for a period of time (i.e., at least 7 days) to classify the type, the size, the position, the direction of all stationary objects in the room (location) using a multi-class classifier. The multi-class classifier is one of, but is not limited to, XGBoost model and decision tree classifier method.

The human wellness recommendation system 103 may also monitor the multiple angular data in a room (location) to identify a physical profile of each user and activity of each user for a period of time i.e., at least 30 days. The human wellness recommendation system 103 uses a reinforcement learning technique to identify the physical profile of each user. The physical profile comprises information associated with user height, user body type, user shape, user age, user gender and stage type, user movement (i.e., gait), user average speed of movement, and restricted user movement. During the training phase, the reinforcement learning technique is provided with feedback from a user to improve the accuracy for the physical profile of each user in the loT environment. In one embodiment, the physical profile comprises breathing signature of each user in the loT environment in addition to the above-mentioned physical profile. Since the physical profile is unique to each person, each physical profile is tagged with an identifier to uniquely identify each person.

The human wellness recommendation system 103 uses a Recurrent Neural Network (RNN) technique based classification to monitor the activity of each user identified in the loT environment. The activity profile of users includes any kind of physical activity, such as walking, moving, running, crawling, sitting, standing, jumping, bending, cleaning, eating, and the like. The physical profile and the activity profile of users are stored in the database 113 by the human wellness recommendation system 103 to be used during the application phase/prediction phase. The physical profile and the activity profile of users stored in the database 113 are referred as historic physical profile and historic activity profile of users.

The human wellness recommendation system 103 also tracks an operational state of one or more loT devices at in each room (location) within the loT environment and corresponding action performed by one or more users to the operational state of one or more loT devices. The loT devices includes, but is not limited to, an air conditioner, a washing machine, a television, a fridge, a vacuum cleaner and the like. The operational state of one or more loT devices refers to functional state of the one or more loT devices, for example, ON state or OFF state. In one embodiment, the operational state of the one or more loT devices further comprises stage of the operational state of the one or more loT devices. For example, when a vacuum cleaner is in the ON state (operation state), the vacuum cleaner may be stationary (stage of the operation state), or the vacuum cleaner may be non-stationary (stage of the operation state) due to performing cleaning operation. During the training phase, the human wellness recommendation system 103 monitors the physical profile of each user, the activity of each user, the operational state of the one or more loT devices, corresponding action performed by each user to the operational state of one or more loT devices, associated location of the one or more loT devices, and a state of environment at the associated location. Thereafter, the human wellness recommendation system 103 correlates the physical profile of each user with at least one of the current activity of each user, the operational state of the one or more loT devices, the corresponding action performed by each users to the operational state of one or more loT devices, the associated location of the one or more loT devices, and the state of environment at the associated location to record as an event. The event is stored in the database 113 as a historic event to be used during the application phase/prediction phase. The correlating of the physical profile of each user with at least one of the current activity of each user, the operational state of the one or more loT devices, the corresponding action performed by each users to the operational state of one or more loT devices, the associated location of the one or more loT devices, and the state of environment at the associated location is performed using a supervised machine learning technique by the human wellness recommendation system 103. The above-mentioned training phase is performed for each room (location) within the loT environment to train the human wellness recommendation system 103.

During the application phase/prediction phase i.e., after the training phase, the human wellness recommendation system 103 identifies the physical profile of each user present in the loT environment using UWB based sensors and historic physical profile stored in the database 113. The physical profile comprises information associated with user height, user body type, user shape, user age, user gender and stage type, user movement (i.e., gait), user average speed of movement, and restricted user movement. The human wellness recommendation system 103 uses multiple angular data received from the UWB based sensors in a time-series manner and the reinforcement learning technique involving feedback from a user of the loT environment to identify the physical profile of each user present in the loT environment. In the next step, the human wellness recommendation system 103 monitors current activity of each user identified in the loT environment using the UWB based sensors and historic activity profile stored in the database 113. The human wellness recommendation system 103 uses the multiple angular data received from the UWB based sensors in a time-series manner and RNN technique based classification to monitor the current activity of each user identified in the loT environment.

In the next operation, the human wellness recommendation system 103 tracks operational state of one or more loT devices at one or more locations within the loT environment.

Thereafter, the human wellness recommendation system 103 predicts a potential anomalous event by correlating the physical profile of each user with the at least one of the current activity of each user, the operational state of the one or more loT devices, the associated location of the one or more loT devices, and a state of environment at the associated location. The potential anomalous event refers to any unexpected event or accident that is likely to occur. Based on the predicted potential anomalous event and a similar historic event identified in the past at a different location within the loT environment and corresponding action performed by the one or more users to the similar historic event, the human wellness recommendation system 103 provides at least one of a risk alert and/or a solution to one or more users at the associated location in the loT environment. The at least one of the risk alert and/or the solution is indicated using at least one IoT enabled device 115 at the associated location in the loT environment. The human wellness recommendation system 103 uses a classification technique to provide the at least one of the risk alert and/or the solution to the one or more user at the associated location in the loT environment. The risk alert refers to an alert that is indicated to one or more users through sound or through light to prevent the one or more users from a possible accident. The solution refers to a solution (message) provided to one or more users through a speaker to prevent any accident from happening.

The human wellness recommendation system 103 determines an action performed by the one or more users on the one or more loT devices at the associated location in the loT environment to the at least one of the risk alert and/or the solution at the associated location. Subsequently, the human wellness recommendation system 103 retrains the correlation between the physical profile of the one or more user with the at least one of the current activity of the one or more user, the operational state of the one or more loT devices, the associated location of the one or more loT devices and the state of environment at the associated location based on the action performed by the one or more users on the one or more loT devices at the associated location in the loT environment.

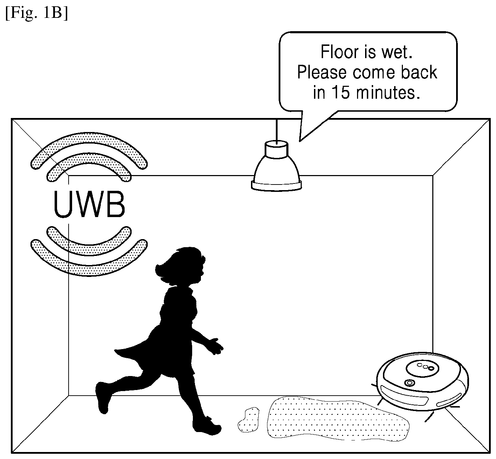

FIG. 1B illustrates an example for providing human wellness recommendation using Ultra-Wideband (UWB) based human activity detection according to an embodiment of the disclosure. In an embodiment, an example for providing human wellness recommendation using Ultra-Wideband (UWB) based human activity detection according to an embodiment of the disclosure. The UWB based sensors 101 obtain multiple angular data in a time-series manner continuously. When the human wellness recommendation system 103 identifies that the woman is walking towards spilled water on the floor in the room, based on multiple angular data, the human wellness recommendation system 103 may turn a speaker to ON state to output the wellness solution "Floor is wet. Please come back in come minutes."

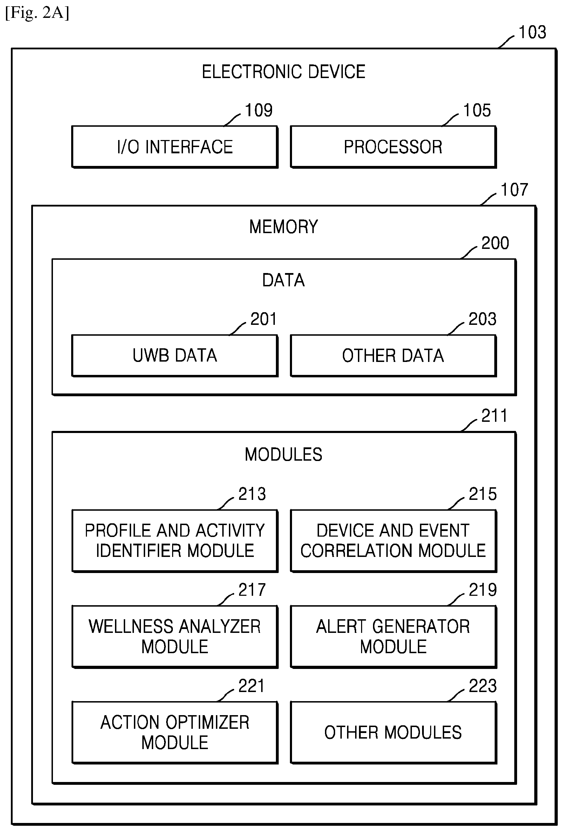

FIG. 2A shows a detailed block diagram of a human wellness recommendation system according to an embodiment of the disclosure.

Referring to FIGS. 2A, the human wellness recommendation system 103, in addition to the I/O interface 109 and the processor 105 described above, includes data 200 and one or more modules 211, which are described below. The data 200 is stored within the memory 107. The data 200 may include UWB data 201 and other data 203.

The UWB data 201 includes multiple angular data collected in a time-series manner and sent by the UWB based sensors 101 to the human wellness recommendation system 103. The multiple angular data comprises properties of scattered signals such as SS, TDA, ToA and AoA.

The other data 203 includes temporary data and temporary files, generated by modules 211 for performing various functions of the human wellness recommendation system 103.

The data 200 in the memory 107 are processed by the one or more modules 211 present within the memory 107 of the human wellness recommendation system 103. In the embodiment, the one or more modules 211 is implemented as dedicated hardware units. As used herein, the term "unit" may refer to an Application Specific Integrated Circuit (ASIC), an electronic circuit, a Field-Programmable Gate Arrays (FPGA), Programmable System-on-Chip (PSoC), a combinational logic circuit, and/or other suitable components that provide the described functionality. In some implementations, the one or more modules 211 are communicatively coupled to the processor 105 for performing one or more functions of the human wellness recommendation system 103. The said modules 211 when configured with the functionality defined in the disclosure results in a novel hardware.

In one implementation, the one or more modules 211 includes, but are not limited to, a profile and activity identifier module 213, a device and event correlation module 215, a wellness analyzer module 217, an alert generator module 219, and an action optimizer module 221. The one or more modules 211 may also include other modules 223 to perform various miscellaneous functionalities of the human wellness recommendation system 103.

For ease of understanding, the operation of the human wellness recommendation system 103 is explained in context of a single room (location) within an IoT environment.

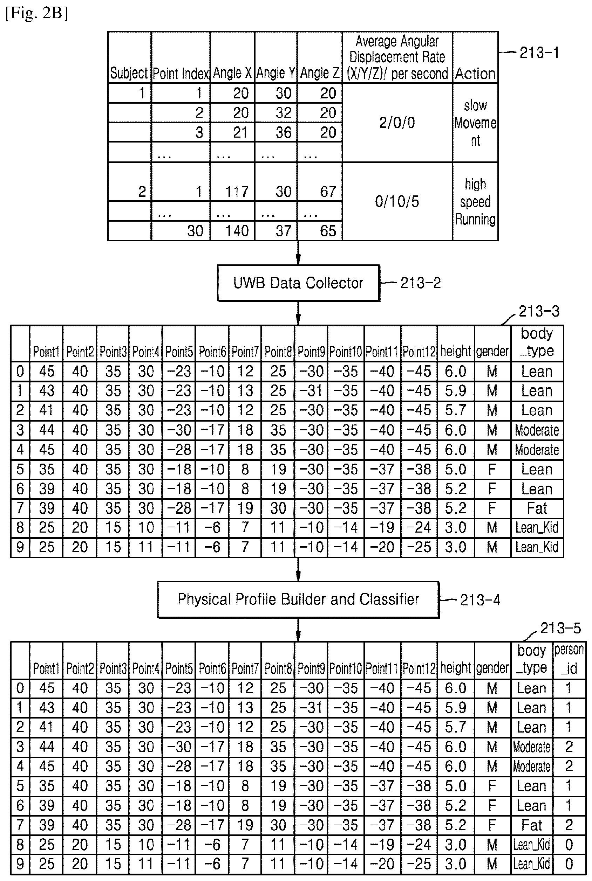

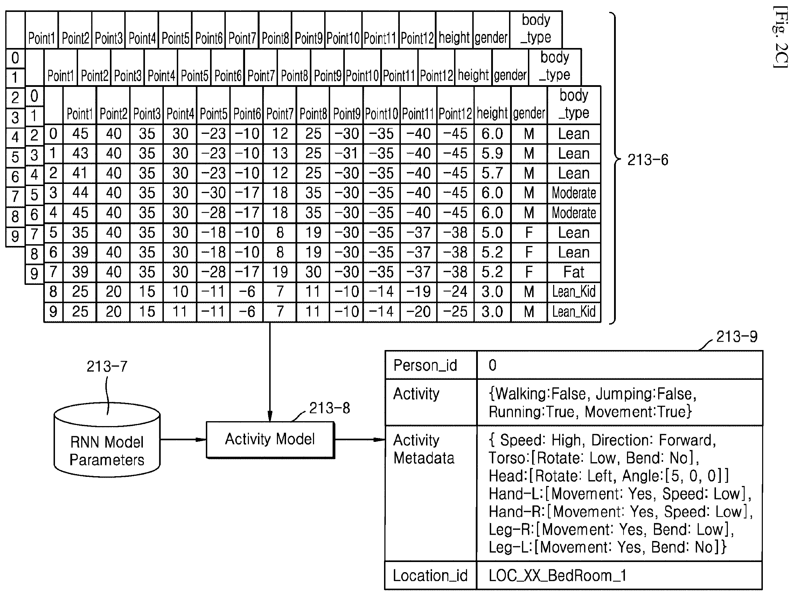

Referring to FIGS. 2B and 2C, the profile and activity identifier module 213 comprises an UWB data collector sub-module (referred as UWB data collector) 213-2 (shown in FIG. 2B), a physical profile builder and classifier sub-module (referred as physical profile builder and classifier) 213-4 (shown in FIG. 2B) and an activity model sub-module (referred as activity model) 213-8 (shown in FIG. 2C). The profile and activity identifier module 213 identifies a physical profile of each user present in an loT environment using UWB based sensors 101 and historic physical profile stored in the database 113, wherein the UWB based sensors 101 are installed in the loT environment.

The profile and activity identifier module 213 monitors current activity of each user identified in the loT environment using the UWB based sensors 101 and historic activity profile stored in the database 113. The UWB based sensors 101 continuously monitor the room (location) within the loT environment and collect multiple angular data 213-1 in a time-series manner. The multiple angular data 213-1 collected in a time-series manner is sent by the UWB based sensors 101 to the profile and activity identifier module 213. The UWB data collector 213-2 of the profile and activity identifier module 213 identifies a physical profile of each user present in the room (location) within the IoT environment using the multiple angular data 213-1 and historic physical profile stored in the database 113. The physical profile and the historic physical profile includes information associated with user height, user body type, user shape, user age, user gender and stage type, user movement, user average speed of movement, and restricted user movement. An example of output of the UWB data collector 213-2 is shown as reference 213-3. The output 213-3 of the UWB data collector 213-2 is passed on to the physical profile builder and classifier 213-4 of the profile and activity identifier module 213. The physical profile builder and classifier 213-4 identifies an identifier assigned to each person using the output 213-3 and the historic physical profile stored in the database 113. The identifier is assigned to each person during the training phase to uniquely identify each person. An example output of the physical profile builder and classifier 213-4 is shown as reference 213-5. The output 213-5 contains person_id (dashed column in FIG. 2B) as an identifier identified by the physical profile builder and classifier 213-4. The output 213-5 contains physical profile along with unique identifier for each user profile. The output 213-5 represents output of the physical profile builder and classifier 213-4 at one instance of time. However, the output of the physical profile builder and classifier 213-4 may include a plurality of output 213-6 (shown in FIG. 2C) produced at successive instances of time (i.e., in a time-series manner). The output 213-6 of the physical profile builder and classifier 213-4 is sent to the activity model 213-8. The profile and activity identifier module 213 uses a reinforcement learning technique involving feedback from a user of the loT environment and the multiple angular data 213-1 collected in a time-series manner received from the UWB based sensors 101 to identify the physical profile of each user present in the room (location) within the loT environment.

Thereafter, the activity model 213-8 receives the plurality of output 213-6 from the physical profile builder and classifier 213-4. Using a RNN technique based classification (also referred as RNN model parameters) 213-7, the plurality of output 213-6 and the historic activity profile stored in the database 113, the activity model 213-8 identifies activity of each user identified in the loT environment. The historic activity profile of user comprises of any kind of physical activity, such as walking, moving, running, crawling, sitting, standing, jumping, bending, cleaning, eating and the like. The RNN technique based classification uses/assigns weights to classify activity of each user identified in the room (location) within the loT environment. The output of the activity model 213-8 is shown as reference 213-9, which represents activity of each user in the room (location) within the loT environment along with along with additional metadata. For instance, the user identified in the room (location) is with person_id (identifier) as 0, activity performed is running and movement, activity metadata describing movement of user's body and location_id being bedroom. The output 213-9 represent activity of a single user. If there are more than one user in the same room (location), in that case the output of the activity model 213-8 is equal to number of users i.e., one output 213-9 for each user in the room (location).

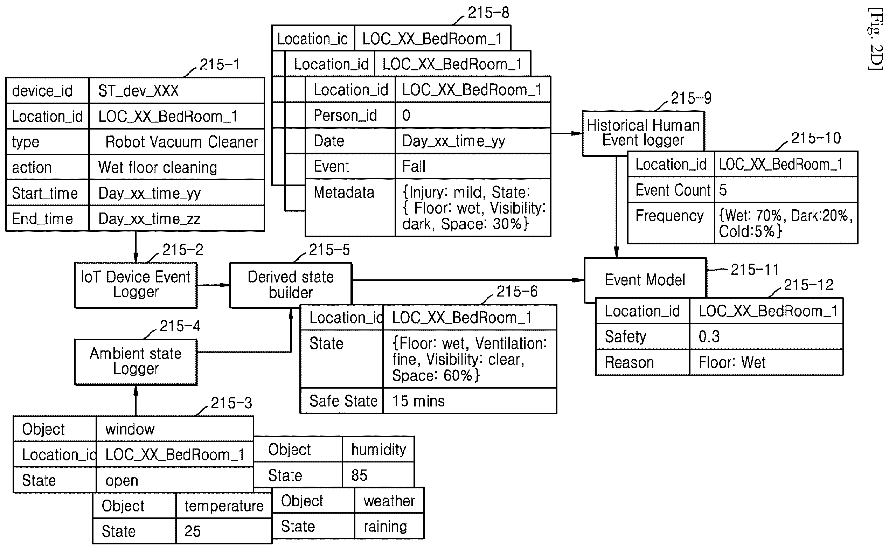

Referring to FIG. 2D, the device and event correlation module 215 comprises of an loT device event logger sub-module (referred as IoT device event logger) 215-2, an ambient state logger sub-module (referred as ambient state logger) 215-4, a derived state builder sub-module (referred as derived state builder) 215-5, a historic human event logger sub-module (referred as historic human event logger) 215-9 and an event model sub-module (referred as event model) 215-11 as shown in FIG. 2D. The device and event correlation module 215 tracks operational state of one or more loT devices at one or more locations within the loT environment. In one embodiment, the device and event correlation module 215 correlates the physical profile of each user with at least one of the current activity of each user, the operational state of the one or more loT devices, associated location of the one or more loT devices, and a state of environment at the associated location.

According to an embodiment, the device and event correlation module 215 along with the wellness analyzer module 217 correlates the physical profile of each user with at least one of the current activity of each user, the operational state of the one or more loT devices, associated location of the one or more loT devices, and a state of environment at the associated location. The correlating the physical profile of each user with the at least one of the current activity of each user, the operational state of the one or more loT devices, the associated location of the one or more loT devices and the state of environment at the associated location is done using a supervised machine learning technique.

The loT device event logger 215-2 of the device and event correlation module 215 identifies operational state (also referred as real time event logs the one or more loT devices) of the one or more loT devices in the room (location) within the loT environment. An example of the operational state of a (robot) vacuum cleaner identified by the loT device event logger 215-2 is shown as reference 215-1. At the same time, the ambient state logger 215-4 of the device and event correlation module 215 identifies ambient state of in the room (location) (also referred as ambient state and contextual parameters) within the loT environment. The ambient state refers to humidity in the room (location), temperature in the room (location), current weather condition, state of windows in in the room (location). An example of the ambient state identified by the ambient state logger 215-4 is shown as reference 215-3. The output of the loT device event logger 215-2 (i.e., the operational state of a (robot) vacuum cleaner) and the output of the ambient state logger 215-4 (i.e., ambient state in the room (location) within the loT environment) are sent to the derived state builder 215-5. The derived state builder 215-5 determines/derives a state of the room (location) (also referred as state of the location based on context). The state comprises current state or status of the room (location) and safety state or status of the room (location). The safety state indicates time required for the room (location) to be safe. An example of the state of the room (location) determined/derived by the derived state builder 215-5 is shown as reference 215-6. The output of the derived state builder 215-5 (i.e., the state of the room (location)) is given to the event model 215-4 as one of the inputs. The other input to the event model 215-4 is received from the historic human event logger 215-9. The historic human event logger 215-9 retrieves/collects similar historic events that are stored in the database 113. The historic events relate to similar events that have occurred in the past in the same room (location) or in a different room (location) within the loT environment and recorded in the database 113 during the training phase or during application phase. An example of the similar historic events that have occurred in the past in the same room (location) along with additional metadata retrieved/collected by the historic human event logger 215-9 is shown as reference 215-8. Using the retrieved/collected similar historic events from the database 113, the historic human event logger 215-9 identifies frequency of similar events in the past and effect of such events on one or more users in the room (location). An example of the frequency of similar events in the past in the room (location) (also referred as event history of the room (location)) identified by the historic human event logger 215-9 is shown as reference 215-10. Thereafter, using the output of the derived state builder 215-5 (i.e., the state of the room (location)) and the output of the historic human event logger 215-9 (i.e., the frequency of similar events in the past and effect of such events on one or more users in the room (location)), the event model 215-11 identifies a probabilistic safety state (between 0.0 and 1.0) with metadata (also, referred as current safe state probability of the location) comprising current time, place and event context of the room (location). An example of the probabilistic safety state identified by the event model 215-11 is shown as reference 215-12.

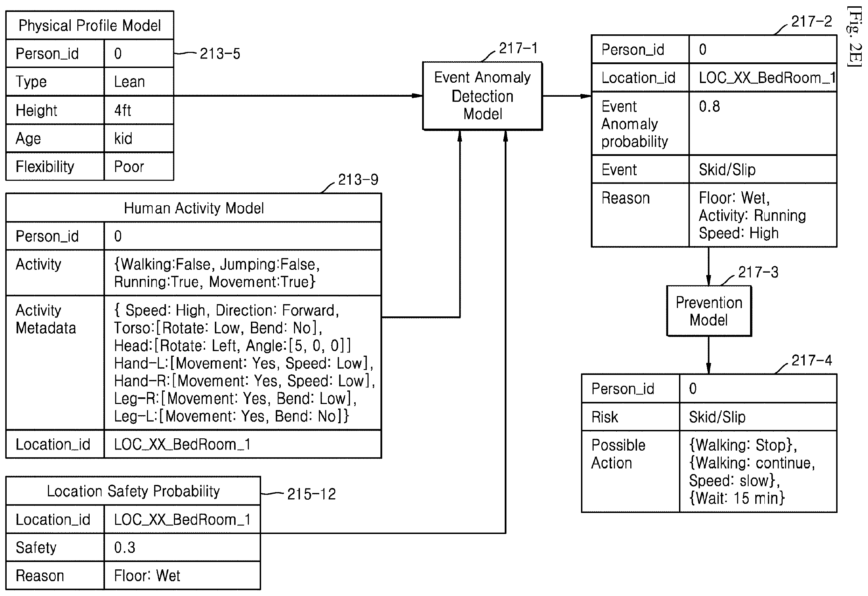

Referring to FIG. 2E, the wellness analyzer module 217 comprises an event anomaly detection model sub-module (referred as event anomaly detection model) 217-1 and a prevention model sub-module (referred as prevention model) 217-3. The wellness analyzer module 217 predicts a potential anomalous event by correlating the physical profile of each user with at least one of the current activity of each user, the operational state of the one or more loT devices, associated location of the one or more loT devices, and a state of environment at the associated location. The event anomaly detection model 217-1 of the wellness analyzer module 217 receives the output of the physical profile builder and classifier 213-4 of the profile and activity identifier module 213 shown as reference 213-5, the output of the activity model 213-8 of the profile and activity identifier module 213 shown as reference 213-9, and the output of the event model 215-11 of the device and event correlation module 215 shown as reference 21512.

Thereafter, the event anomaly detection model 217-1 correlates the outputs from the physical profile builder and classifier 213-4, the activity model 213-8, and the event model 215-11 to determine probability of an anomalous event from occurring in the room (location) in the loT environment. In one embodiment, the event anomaly detection model 217-1 uses RNN technique with Softmax function for performing correlation. An example of the probability of an anomalous event (also referred as probability of risk of event) determined by the event anomaly detection model 217-1 is shown as reference 217-2. The output of the event anomaly detection model 217-1 (i.e., probability of an anomalous event) is given to the prevention model 217-3 of the wellness analyzer module 217, which determines possible action for safety of the user in the room (location). In one embodiment, the prevention model 217-3 uses an Adaptive Boosting (AdaBoost) technique for determining possible action for safety of the user. An example of the possible action for safety (also referred as identified set of actions that can be taken to avoid risk) determined by the prevention model 217-3 is shown as reference 217-4.

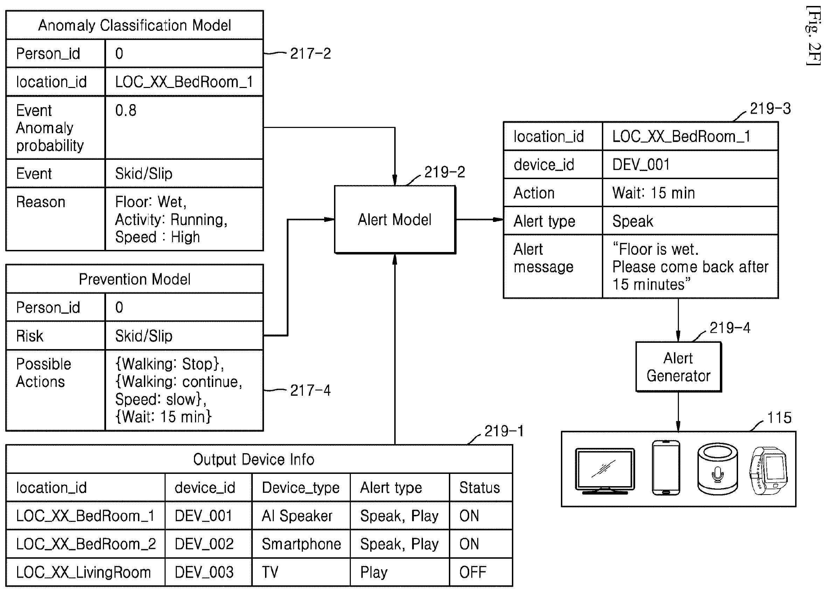

Referring to FIG. 2F, the alert generator module 219 comprises an alert model sub-module (referred as alert model) 219-2 and an alert generator sub-module (referred as alert generator) 219-4 as shown in. The alert generator module 219 provides at least one of a wellness risk alert and/or a wellness solution to one or more users at the associated location in the loT environment based on the predicted potential anomalous event and similar historic event identified in past at different location within the loT environment and corresponding action performed by the one or more users to the similar historic event. The alert model 219-2 of the alert generator module 219 receives the output of the event anomaly detection model 217-1 of the wellness analyzer module 217 shown as reference 217-2, the output of the prevention model 217-3 of the wellness analyzer module 217 shown as reference 217-4, and device information (also referred as available IoT enabled devices and their status) of at least one IoT enabled device 115 in the room (location) in the loT environment from the at least one IoT enabled device 115 in the room shown as reference 219-1.

Thereafter, the alert model 219-2 determines at least one of a wellness risk alert and/or a wellness solution to be provided to the one or more users in the room (location) in the loT environment based on the outputs from the event anomaly detection model 217-1, the prevention model 217-3, and the device information of the at least one IoT enabled device 115 in the room (location) in the loT environment. An example of the at least one of a wellness risk alert and/or a wellness solution determined by the alert model 219-2 is shown as reference 219-3. In one embodiment, the alert model 219-2 uses RNN technique based classification for determining at least one of a wellness risk alert and/or a wellness solution. The output of the alert model 219-2 (i.e., at least one of a wellness risk alert and/or a wellness solution) is given to the alert generator 219-4 of the alert generator module 219, which provides the at least one of a wellness risk alert and/or a wellness solution to the at least one IoT enabled device 115 in the room (location) in the loT environment. The alert generator module 219 uses a classification technique to determine and provide the at least one of a wellness risk alert and/or a wellness solution.

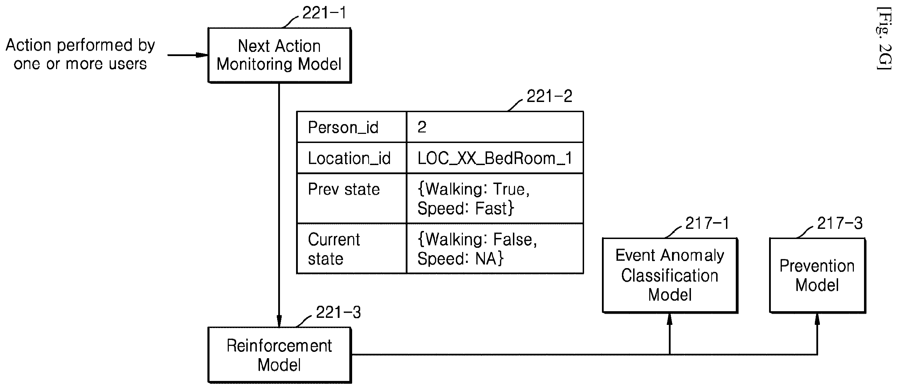

Referring to FIG. 2G, the action optimizer module 221 comprises of a next action monitoring model sub-module (referred as next action monitoring model) 221-1 and a reinforcement model sub-module (referred as reinforcement model) 221-3 as shown in. The action optimizer module 221 determines action performed by the one or more users on the one or more loT devices at the associated location in the loT environment to the at least one of the wellness risk alert and/or the wellness solution at the associated location. Thereafter, the action optimizer module 221 retrains the correlation between the physical profile of the one or more user with at least one of the current activity of the one or more user, the operational state of the one or more loT devices, the associated location of the one or more loT devices and the state of environment at the associated location based on the action performed by the one or more users on the one or more loT devices at the associated location in the loT environment.

In detail, the next action monitoring model 221-1 of the action optimizer module 221 determines/monitors the action performed by the one or more users on the one or more loT devices 115 in the room (location) in the loT environment to the at least one of the wellness risk alert and/or the wellness solution provided in the room (location). An example of the action performed by the one or more users (also referred as observed user action based on alert) to the at least one of the wellness risk alert and/or the wellness solution determined/monitored by the next action monitoring model 221-1 is shown as reference 221-2.

Thereafter, the reinforcement model 221-3 receives the output of the next action monitoring model 221-1 (i.e., the action performed by the one or more users). Based on the output of the next action monitoring model 221-1, the reinforcement model 221-3 retrains the event anomaly detection model 217-1 and the prevention model 217-3 of the wellness analyzer module 217 to improve the correlation between the physical profile of the one or more user, the current activity of the one or more user, the operational state of the one or more loT devices, the room (location) of the one or more loT devices, and the state of environment in the room (location). The reinforcement model 221-3 checks if there is a deviation of more than a predefined threshold, for example 20%, in the action performed by the one or more users to the at least one of the wellness risk alert and/or the wellness solution provided in the room (location). If the deviation is more than the predefined threshold, then the reinforcement model 221-3 considers as the wellness risk alert and/or the wellness solution as a failed wellness risk alert and/or wellness solution. The reinforcement model 221-3 penalizes the event anomaly detection model 217-1 and the prevention model 217-3 by negative weight adjustment. As an example, if in multiple occasions a user is suggested to stop movement in the room (location) and the user does not comply, then that situation i.e., movement of the user for those occasions is considered to be not an anomalous situation in that room (location). If the deviation is less than the predefined threshold, then the reinforcement model 221-3 considers as the wellness risk alert and/or the wellness solution as a successful wellness risk alert and/or wellness solution. The reinforcement model 221-3 awards the event anomaly detection model 217-1 and the prevention model 217-3 by positive weight adjustment.

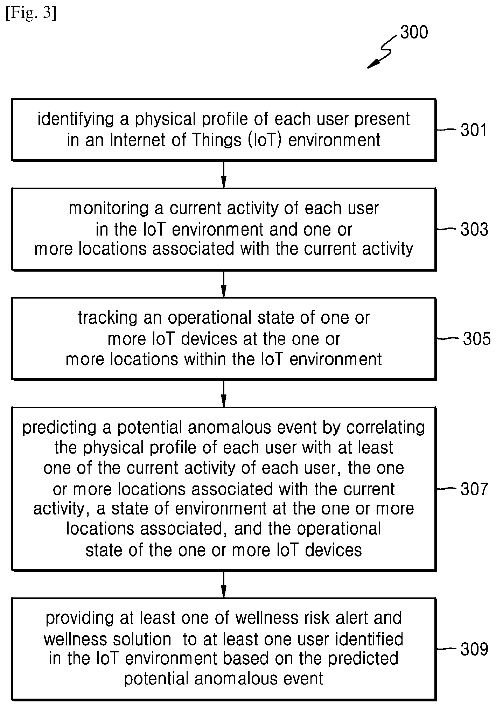

FIG. 3 shows a flowchart illustrating a method for providing human wellness recommendation using UWB based human activity detection according to an embodiment of the disclosure.

Referring to FIG. 3, the method 300 includes one or more operations for providing human wellness recommendation using UWB based human activity detection in accordance with some embodiments of the disclosure. The method 300 may be described in the general context of computer executable instructions. Generally, computer executable instructions can include routines, programs, objects, components, data structures, procedures, units, and functions, which perform particular functions or implement particular abstract data types.

The method can be implemented in any suitable hardware, software, firmware, or combination thereof.

At operation 301, the profile and activity identifier module 213 identifies a physical profile of each user present in an loT environment using UWB based sensors and historic physical profile stored in a database. The UWB based sensors may be installed in the loT environment. The identifying of the physical profile of each user present in the loT environment may be performed using multiple angular data received from the UWB based sensors in a time-series manner and using a reinforcement learning technique involving feedback from a user of the loT environment. The physical profile and the historic physical profile may comprise information associated with user height, user body type, user shape, user age, user gender and stage type, user movement, user average speed of movement, and restricted user movement.

At operation 303, the profile and activity identifier module 213 monitors a current activity of each user in the loT environment and one or more locations associated with the current activity. The profile and activity identifier module 213 may monitor at least one of current activity of each user in the loT environment using the UWB based sensors and historic activity profile stored in the database. The monitoring of the current activity of each user identified in the loT environment may be performed using multiple angular data received from the UWB based sensors in a time-series manner and using Recurrent Neural Network (RNN) technique based classification.

At operation 305, the device and event correlation module 215 tracks an operational state of one or more loT devices at the one or more locations within the loT environment. The device and event correlation module 215 tracks operational state of one or more IoT devices at one or more locations within the loT environment.

At operation 307, the wellness analyzer module 217 predicts a potential anomalous event by correlating the physical profile of each user with at least one of the current activity of each user, the one or more locations associated with the current activity, a state of environment at the one or more locations associated, and the operational state of the one or more loT devices. The wellness analyzer module 217 may predict a potential anomalous event by correlating the physical profile of each user with at least one of the current activity of each user, the operational state of the one or more loT devices, associated location of the one or more loT devices, or a state of environment at the associated location. The correlating of the physical profile of each user with at least one of the current activity of each user, the operational state of the one or more loT devices, the associated location of the one or more loT devices, and the state of environment at the associated location may be performed using a supervised machine learning technique.

At operation 309, the alert generator module 219 provides at least one of risk alert and/or solution to at least one user identified in the loT environment based on the predicted potential anomalous event. The alert generator module 219 provides at least one of a risk alert and/or a solution to one or more users at the associated location in the loT environment based on the predicted potential anomalous event and a similar historic event identified in the past at a different location within the loT environment and corresponding action performed by the one or more users to the similar historic event. The providing at least one of the risk alert and the solution to the one or more user at the associated location in the loT environment may be performed using a classification technique. The at least one of the risk alert and/or the solution may be indicated using at least one IoT device 115 present at the one or more locations in the loT environment.

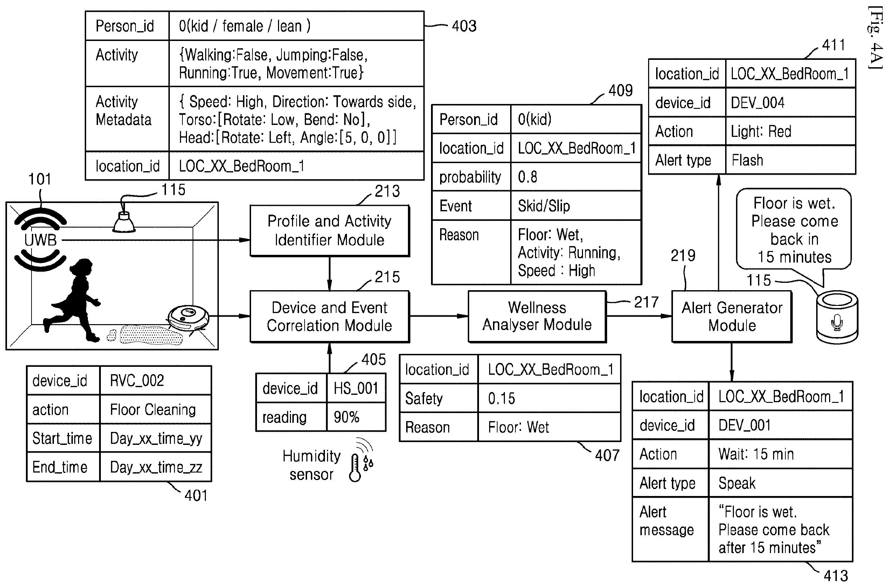

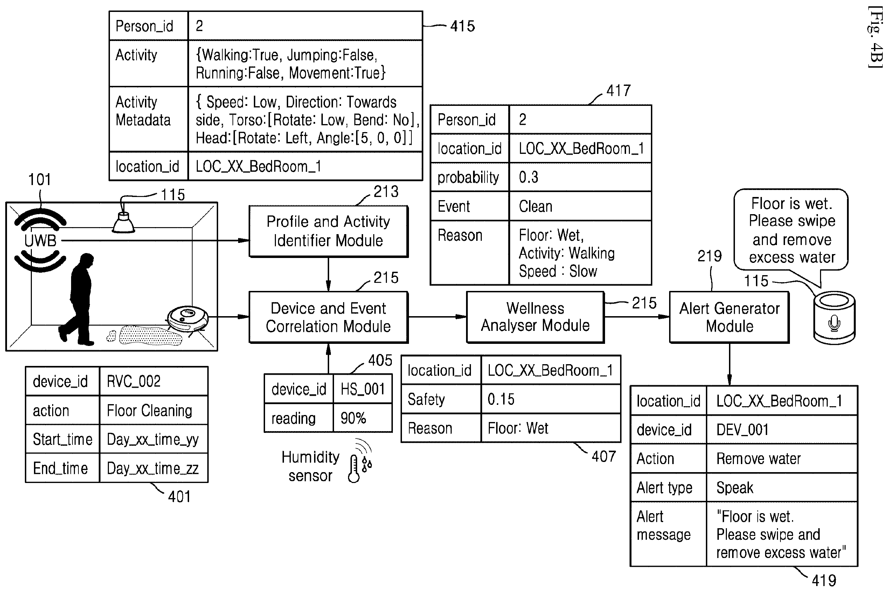

FIGS. 4A and 4B illustrate a first example for providing human wellness recommendation based on Ultra-Wideband (UWB) based human activity detection according to various embodiments of the disclosure.

Referring to FIG. 4A, consider a scenario where a girl (user) enters a room by running and vacuum cleaner is cleaning a wet floor in the room due to spilled water on a floor. The UWB based sensors 101 collect multiple angular data in a time-series manner continuously and send the collected multiple angular data to the human wellness recommendation system 103. When the girl enters the room by running, the profile and activity identifier module 213 of the human wellness recommendation system 103 identifies a physical profile of the girl present in the room using UWB based sensors and historic physical profile stored in the database 113. Thereafter, the profile and activity identifier module 213 monitors activity of the girl identified in the room using the UWB based sensors 101 and historic activity profile stored in the database 113. In this case, the profile and activity identifier module 213 identifies that the girl is running towards spilled water on the floor in the room. The output 403 of the profile and activity identifier module 213 is sent to the device and event correlation module 215. The device and event correlation module 215 of the human wellness recommendation system 103 tracks an operational state of one or more loT devices in the room. In this case, the device and event correlation module 215 tracks the operational state of the (robot) vacuum cleaner 401 in the room and ambient state (e.g., humidity in the room) 405 of the room. Using the output 403 of the profile and activity identifier module 213, the operational state of the (robot) vacuum cleaner 401 in the room and the ambient state (e.g., humidity in the room) 405 of the room, the device and event correlation module 215 correlates the physical profile of the girl, with at least one of the current activity of the girl, the operational state of the (robot) vacuum cleaner, room (i.e., associated location of the (robot) vacuum cleaner), and the humidity (i.e., a state of environment) at the room.

The wellness analyser 217 of the human wellness recommendation system 103 predicts a potential anomalous event 409 using the correlated output 407 from the device and event correlation module 215. In this case, using the surrounding awareness that water spillage in the room and girls' history of skidding on a split water when running, the wellness analyser 217 predicts a potential anomalous event 409 (i.e., possibility of skidding event) of the girl in the room. Thereafter, the alert generator 219 of the human wellness recommendation system 103 provides at least one of a risk alert 411 and a solution 413 to the girl in the room based on the predicted potential anomalous event 409 and a similar historic event identified in the past at a different location within the loT environment and corresponding action performed by the girl to the similar historic event. In this case, the alert generator 219 turns the light bulb 115 red (an indication of risk alert) 411 and turns the speaker 115 to ON state to play the solution 413 "Floor is wet. Please come back in 15 mins"

Referring to FIG. 4B, consider a scenario where a woman (user) enters a room by walking and vacuum cleaner is cleaning a wet floor in the room due to spilled water on a floor. The UWB based sensors 101 collect multiple angular data in a time-series manner continuously and send the collected multiple angular data to the human wellness recommendation system 103. When the woman enters the room by walking, the profile and activity identifier module 213 of the human wellness recommendation system 103 identifies a physical profile of the woman present in the room using UWB based sensors and historic physical profile stored in the database 113. Thereafter, the profile and activity identifier module 213 monitors activity of the woman identified in the room using the UWB based sensors 101 and historic activity profile stored in the database 113. In this case, the profile and activity identifier module 213 identifies that the woman is walking towards spilled water on the floor in the room. The output 415 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the (robot) vacuum cleaner 401 in the room and ambient state (e.g., humidity in the room) 405 of the room. Using the output 415 of the profile and activity identifier module 213, the operational state of the (robot) vacuum cleaner 401 in the room and the ambient state (e.g., humidity in the room) 405 of the room, the device and event correlation module 215 correlates the physical profile of the woman with at least one of the current activity of the woman, the operational state of the (robot) vacuum cleaner, room (i.e., associated location of the (robot) vacuum cleaner), and the humidity (i.e., a state of environment) at the room.

The wellness analyser 217 of the human wellness recommendation system 103 predicts a potential anomalous event 409 using the correlated output 407 from the device and event correlation module 215. In this case, using the surrounding awareness that water spillage in the room and woman' history of cleaning water spillage multiple times in different location in the loT environment, the wellness analyser 217 predicts a potential anomalous event 417 (i.e., possibility of cleaning event) of the woman in the room. Thereafter, the alert generator 219 of the human wellness recommendation system 103 provides a wellness solution 419 to the woman in the room based on the predicted potential anomalous event 417 and similar historic event identified in past at different location within the loT environment and corresponding action performed by the woman to the similar historic event. In this case, the alert generator 219 turns the speaker 115 to ON state to play the wellness solution 419 "Floor is wet. Please swipe and remove excess water"

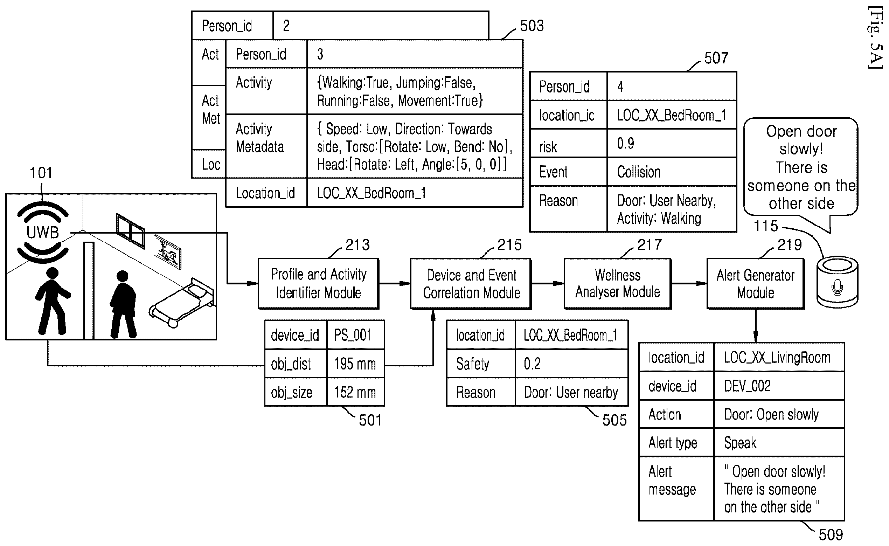

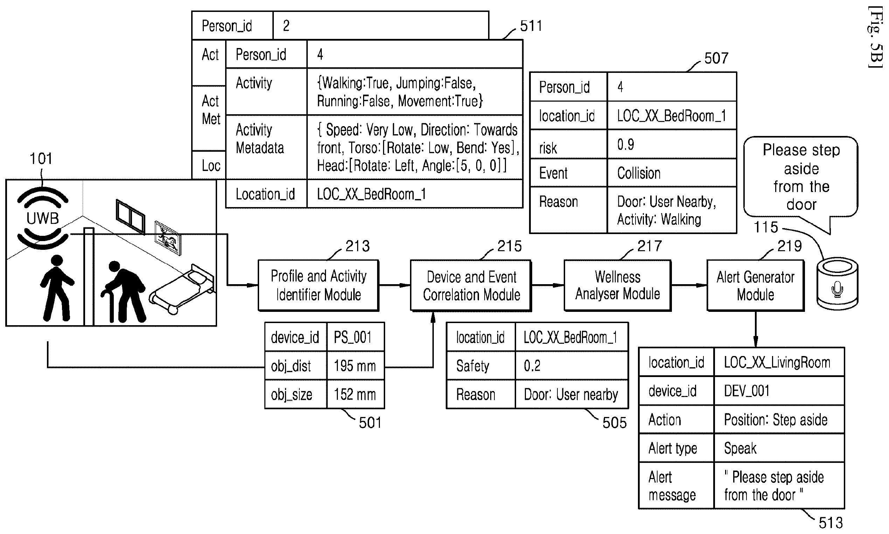

FIGS. 5A and 5B illustrate second example for providing human wellness recommendation based on Ultra-Wideband (UWB) based human activity detection according to various embodiments of the disclosure.

Referring to FIG. 5A, consider a scenario where two adults (users) with normal physical profiles are approaching a same (closed) door from either side of a different rooms. The UWB based sensors 101 collect multiple angular data in a time-series manner continuously and send the collected multiple angular data to the human wellness recommendation system 103. When the two adults are approaching the same door from either side of the different rooms, the profile and activity identifier module 213 of the human wellness recommendation system 103 identifies a physical profile of the two adults in each room using UWB based sensors and historic physical profile stored in the database 113. Thereafter, the profile and activity identifier module 213 monitors activity of the two adults using the UWB based sensors 101 and historic activity profile stored in the database 113. In this case, the profile and activity identifier module 213 identifies that the two adults are approaching the same door from different sides. The output 503 of the profile and activity identifier module 213 is sent to the device and event correlation module 215. The device and event correlation module 215 of the human wellness recommendation system 103 tracks operational state of one or more loT devices in the room. In this case, the device and event correlation module 215 uses the UWB based sensors 101 in the room to calculate the distance of two adults from the door. Using the output 503 of the profile and activity identifier module 213 and the calculated distance 501, the device and event correlation module 215 correlates the physical profile of the two adults with at least one of the current activity of the two adults, the calculated distance 501, and rooms (i.e., associated locations of the UWB based sensors 101).

The wellness analyser 217 of the human wellness recommendation system 103 predicts a potential anomalous event 507 using the correlated output 505 from the device and event correlation module 215. In this case, using the surrounding awareness that two adults are approaching the same (closed) door and the two adults' history of capable of fast movement in the loT environment, the wellness analyser 217 predicts a potential anomalous event 507 (i.e., possibility of collision event) between the two adults approaching the same door. Thereafter, the alert generator 219 of the human wellness recommendation system 103 provides at least one of a risk alert 509 and a solution to the two adults based on the predicted potential anomalous event 507 and a similar historic event identified in the past at a different location within the loT environment and corresponding action performed by the two adults to the similar historic event. In this case, the alert generator 219 turns the alarm (buzzer) 115 to ON state (an indication of wellness risk alert) 509 and turns the speaker 115 to ON state to play the solution "Please step aside from the door"

Referring to FIG. 5B, consider a scenario where two adults (users) one with normal physical profile and other with weak physical profile are approaching the same (closed) door from different sides. The UWB based sensors 101 collect multiple angular data in a time-series manner continuously and send the collected multiple angular data to the human wellness recommendation system 103. When the two adults are approaching the same door from different sides, the profile and activity identifier module 213 of the human wellness recommendation system 103 identifies a physical profile of the two adults in the either room using UWB based sensors and historic physical profile stored in the database 113. Thereafter, the profile and activity identifier module 213 monitors activity of the two adults using the UWB based sensors 101 and historic activity profile stored in the database 113. In this case, the profile and activity identifier module 213 identifies that the two adults are approaching the same door from either side of the different rooms. The output 511 of the profile and activity identifier module 213 is sent to the device and event correlation module 215. The device and event correlation module 215 of the human wellness recommendation system 103 tracks operational state of one or more loT devices in the room. In this case, the device and event correlation module 215 uses the UWB based sensors 101 in the room to calculate the distance of two adults from the door. Using the output 511 of the profile and activity identifier module 213 and the calculated distance 501, the device and event correlation module 215 correlates the physical profile of the two adults with at least one of the current activity of the two adults, the calculated distance 501, and rooms (i.e., associated locations of the UWB based sensors 101).

The wellness analyser 217 of the human wellness recommendation system 103 predicts a potential anomalous event 507 using the correlated output 505 from the device and event correlation module 215. In this case, using the surrounding awareness that two adults are approaching the same (closed) door and two adults' history i.e., one adult capable of fast movement and other adult capable of slow movement in the loT environment, the wellness analyser 217 predicts a potential anomalous event 507 (i.e., possibility of collision event) between the two adults approaching the same door. Thereafter, the alert generator 219 of the human wellness recommendation system 103 provides a solution 507 to the two adults based on the predicted potential anomalous event 513 and a similar historic event identified in the past at a different location within the loT environment and corresponding action performed by the two adults to the similar historic event. In this case, the alert generator 219 turns the speaker 115 to ON state to output the solution 513 "Open door slowly. There is someone on the other side"

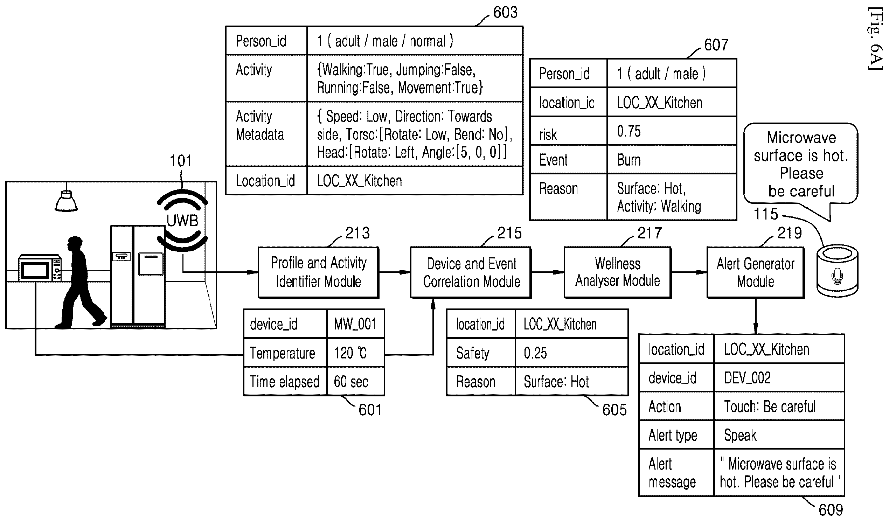

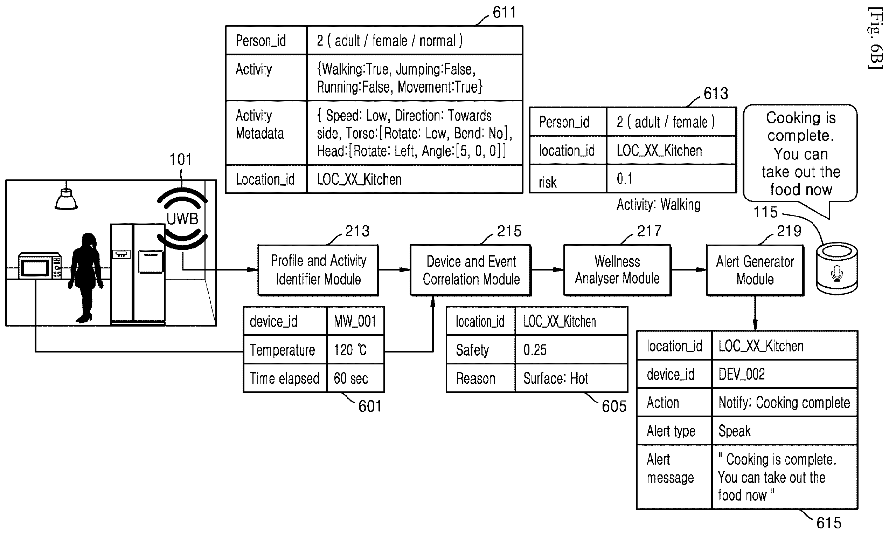

FIGS. 6A and 6B illustrate a third example for providing human wellness recommendation using UWB based human activity detection according to various embodiments of the disclosure.

Referring to FIG. 6A, consider a scenario in a room (kitchen) where an adult female (wife) is cooking food in the oven and an adult male (husband, also referred as user) is walking towards the oven. The UWB based sensors 101 collect multiple angular data in a time-series manner continuously and send the collected multiple angular data to the human wellness recommendation system 103. When the adult male (husband) walks towards the oven, the profile and activity identifier module 213 of the human wellness recommendation system 103 identifies a physical profile of the adult male present in the room using UWB based sensors and historic physical profile stored in the database 113.

Thereafter, the profile and activity identifier module 213 monitors activity of the adult male identified in the room using the UWB based sensors 101 and historic activity profile stored in the database 113. In this case, the profile and activity identifier module 213 identifies that the adult male is walking towards the oven in the room. The output 603 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the oven 601 in the room. Using the output 603 of the profile and activity identifier module 213 and the operational state of the oven 601 in the room, the device and event correlation module 215 correlates the physical profile of the adult male with the at least one of the current activity of the adult male, the operational state of the oven, and room (i.e., associated location of the oven). The wellness analyser 217 of the human wellness recommendation system 103 predicts a potential anomalous event 607 using the correlated output 605 from the device and event correlation module 215. In this case, using the surrounding awareness that adult male is about to touch hot surface, the wellness analyser 217 predicts a potential anomalous event 607 (i.e., possibility of getting burn) of the adult male in the room.

Thereafter, the alert generator 219 of the human wellness recommendation system 103 provides a wellness solution 609 to the adult male in the room based on the predicted potential anomalous event 607 and similar historic event identified in past at different location within the loT environment and corresponding action performed by the adult male to the similar historic event. In this case, the alert generator 219 turns the speaker 115 to ON state to output the wellness solution 609 "Microwave surface is hot. Please be careful"

Referring to FIG. 6B, consider a scenario in a room (kitchen) where an adult female (wife, also referred as user) is cooking food in an oven and the adult female is walking towards the oven. The UWB based sensors 101 collect multiple angular data in a time-series manner continuously and send the collected multiple angular data to the human wellness recommendation system 103. When the adult female walks towards the oven, the profile and activity identifier module 213 of the human wellness recommendation system 103 identifies a physical profile of the adult female present in the room using UWB based sensors and historic physical profile stored in the database 113.

Thereafter, the profile and activity identifier module 213 monitors activity of the adult female identified in the room using the UWB based sensors 101 and historic activity profile stored in the database 113. In this case, the profile and activity identifier module 213 identifies that the adult female is walking towards the oven in the room. The output 611 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the oven 601 in the room. Using the output 611 of the profile and activity identifier module 213 and the operational state of the oven 601 in the room, the device and event correlation module 215 correlates the physical profile of the adult female with at least one of the current activity of the adult female, the operational state of the oven, and room (i.e., associated location of the oven). The wellness analyser 217 of the human wellness recommendation system 103 predicts a potential anomalous event 613 using the correlated output 605 from the device and event correlation module 215. In this case, using the surrounding awareness that adult female is about to touch hot surface, the wellness analyser 217 predicts a potential anomalous event 613 (i.e., possibility of notifying that cooking is complete) of the adult female in the room.

Thereafter, the alert generator 219 of the human wellness recommendation system 103 provides a wellness solution 615 to the adult female in the room based on the predicted potential anomalous event 613 and similar historic event identified in past at different location within the loT environment and corresponding action performed by the adult female to the similar historic event. In this case, the alert generator 219 turns the speaker 115 to ON state to play the wellness solution 615 "Cooking is complete. You can take out the food now"

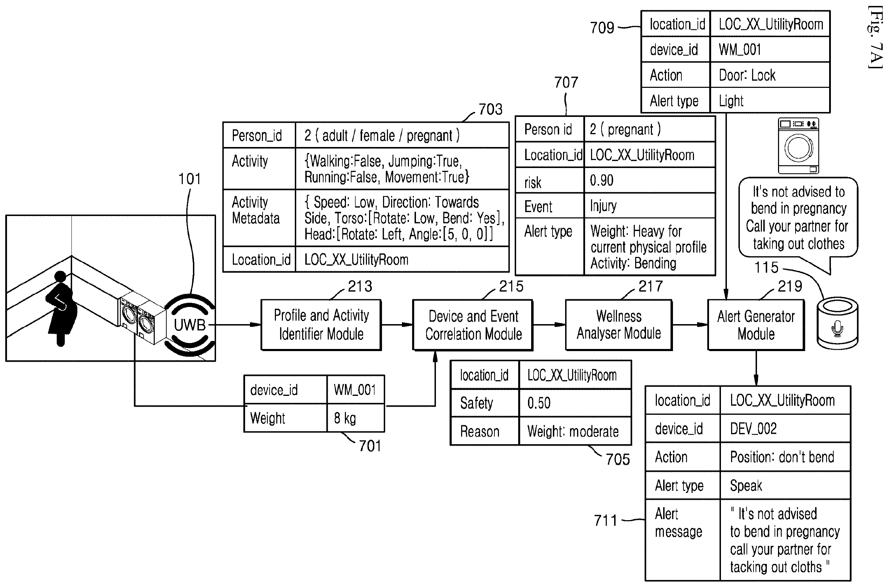

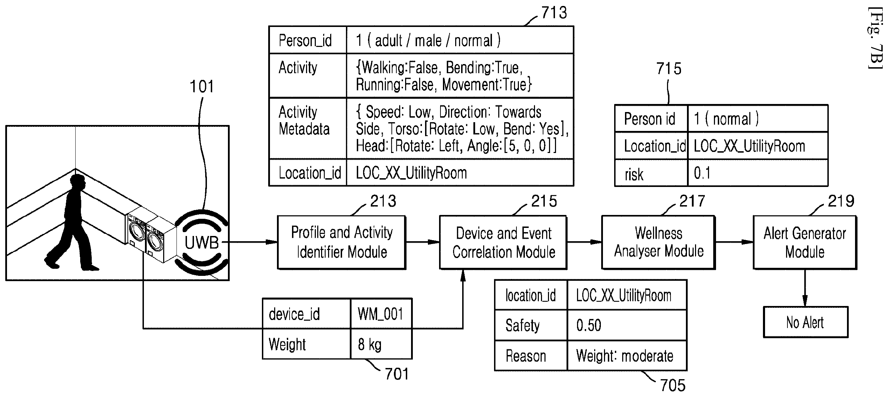

FIGS. 7A and 7B illustrate a fourth example for providing human wellness recommendation using UWB based human activity detection according to various embodiments of the disclosure.

Referring to FIG. 7A, consider a scenario in a room where clothes in a washing machine are ready to be taken out and a pregnant woman (user) is walking towards the washing machine. The UWB based sensors 101 collect multiple angular data in a time-series manner continuously and send the collected multiple angular data to the human wellness recommendation system 103. When the pregnant woman walks towards the washing machine, the profile and activity identifier module 213 of the human wellness recommendation system 103 identifies a physical profile of the pregnant woman present in the room using UWB based sensors and historic physical profile stored in the database 113.

Thereafter, the profile and activity identifier module 213 monitors activity of the pregnant woman identified in the room using the UWB based sensors 101 and historic activity profile stored in the database 113. In this case, the profile and activity identifier module 213 identifies that the pregnant woman is walking towards the washing machine in the room. The output 703 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the washing machine 701 in the room. Using the output 703 of the profile and activity identifier module 213 and the operational state of the washing machine 701 in the room, the device and event correlation module 215 correlates the physical profile of the pregnant woman with at least one of the activity of the pregnant woman, the operational state of the washing machine, and room (i.e., associated location of the washing machine). The wellness analyser 217 of the human wellness recommendation system 103 predicts a potential anomalous event 707 using the correlated output 705 from the device and event correlation module 215. In this case, using the surrounding awareness that weight of clothes is moderate, the wellness analyser 217 predicts a potential anomalous event 707 (i.e., possibility of getting injured due to bending for getting clothes out of the washing machine) of the pregnant woman in the room.

Thereafter, the alert generator 219 of the human wellness recommendation system 103 provides at least one of a wellness risk alert 709 and a wellness solution 711 to the pregnant woman based on the predicted potential anomalous event 707 and similar historic event identified in past at different location within the loT environment and corresponding action performed by the pregnant woman to the similar historic event. In this case, the alert generator 219 turns the light red on the washing machine (an indication of wellness risk alert) 709 and turns the speaker 115 to ON state to output the wellness solution 711 "It's not advised to bend in pregnancy, call your partner for taking out clothes"

Referring to FIG. 7B, consider a scenario in a room where clothes in a washing machine are ready to be taken out and an adult man (user) is walking towards the washing machine. The UWB based sensors 101 collect multiple angular data in a time-series manner continuously and send the collected multiple angular data to the human wellness recommendation system 103. When the adult man walks towards the washing machine, the profile and activity identifier module 213 of the human wellness recommendation system 103 identifies a physical profile of the adult man present in the room using UWB based sensors and historic physical profile stored in the database 113.

Thereafter, the profile and activity identifier module 213 monitors activity of the adult man identified in the room using the UWB based sensors 101 and historic activity profile stored in the database 113. In this case, the profile and activity identifier module 213 identifies that the adult man is walking towards the washing machine in the room. The output 713 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the washing machine 701 in the room. Using the output 713 of the profile and activity identifier module 213 and the operational state of the washing machine 701 in the room, the device and event correlation module 215 correlates the physical profile of the adult man with at least one of the current activity of the adult man, the operational state of the washing machine, and room (i.e., associated location of the washing machine). The wellness analyser 217 of the human wellness recommendation system 103 predicts a potential anomalous event 715 using the correlated output 705 from the device and event correlation module 215. In this case, using the surrounding awareness that weight of clothes is moderate, the wellness analyser 217 predicts no potential anomalous event (i.e., the adult man is capable of getting clothes out of the washing machine) of the adult man in the room.

Thereafter, the alert generator 219 of the human wellness recommendation system 103 provides no wellness risk alert or wellness solution to the adult man based on the absence of any predicted potential anomalous event and similar historic event identified in past at different location within the loT environment and corresponding action performed by the adult man to the similar historic event. In this case, the alert generator 219 presents no alert to the adult man.

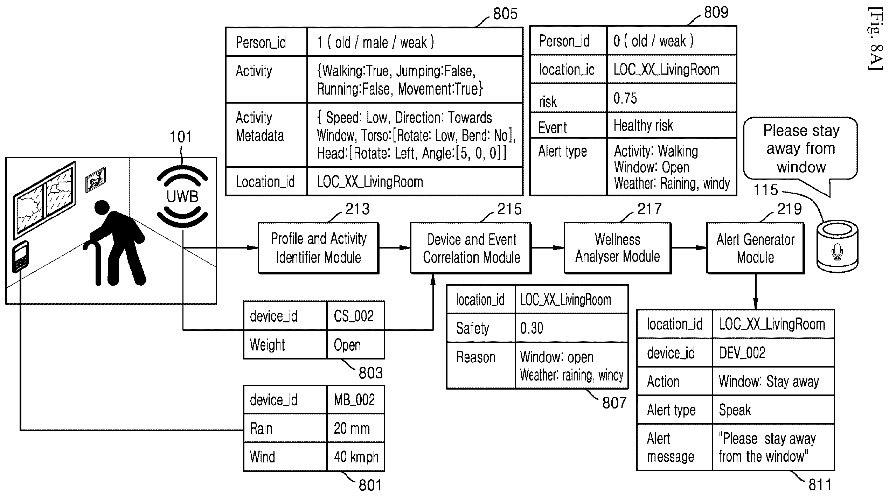

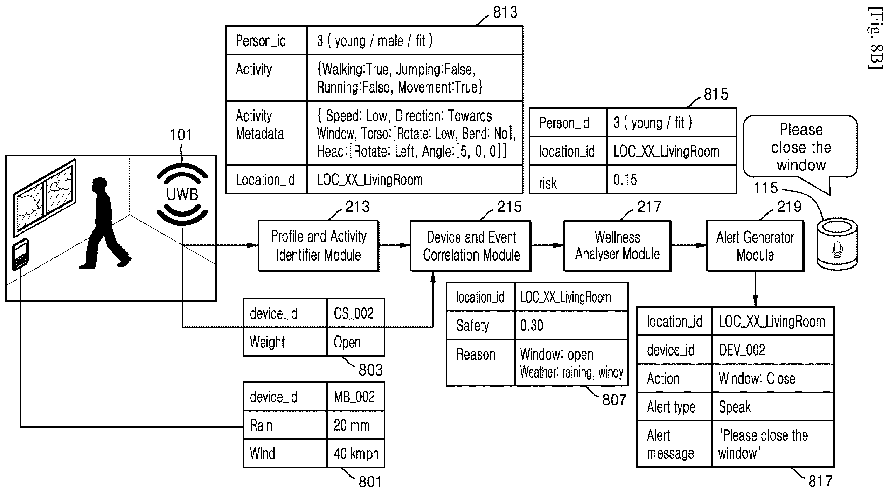

FIGS. 8A and 8B illustrate fifth example for providing human wellness recommendation using UWB based human activity detection according to various embodiments of the disclosure.

Referring to FIG. 8A, consider a scenario in a room where a window is open, and an old man (user) is walking towards the window. The UWB based sensors 101 collect multiple angular data in a time-series manner continuously and send the collected multiple angular data to the human wellness recommendation system 103. When the old man walks towards the window, the profile and activity identifier module 213 of the human wellness recommendation system 103 identifies a physical profile of the old man present in the room using UWB based sensors and historic physical profile stored in the database 113.

Thereafter, the profile and activity identifier module 213 monitors activity of the old man identified in the room using the UWB based sensors 101 and historic activity profile stored in the database 113. In this case, the profile and activity identifier module 213 identifies that the old man is walking towards the window in the room. The output 805 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the weather sensor 801 in the room. Using the output 805 of the profile and activity identifier module 213, an output 803 of the UWB based sensors 101 and the operational state of the weather sensor 801 in the room, the device and event correlation module 215 correlates the physical profile of the old man with at least one of the current activity of the old man, the operational state of the weather sensor, the output of the UWB based sensors 101, and room (i.e., associated location of the weather sensor). The wellness analyser 217 of the human wellness recommendation system 103 predicts a potential anomalous event 809 using the correlated output 807 from the device and event correlation module 215. In this case, using the surrounding awareness that window is open and adverse weather condition, the wellness analyser 217 predicts a potential anomalous event 809 (i.e., possibility of health risk) of the old man in the room.

Thereafter, the alert generator 219 of the human wellness recommendation system 103 provides a wellness solution 811 to the old man based on the predicted potential anomalous event 809 and similar historic event identified in past at different location within the loT environment and corresponding action performed by the old man to the similar historic event. In this case, the alert generator 219 turns the speaker 115 to ON state to play the wellness solution 811 "Please stay away from window"

Referring to FIG. 8B, consider a scenario in a room where a window is open, and a young man (user) is walking towards the window. The UWB based sensors 101 collect multiple angular data in a time-series manner continuously and send the collected multiple angular data to the human wellness recommendation system 103. When the young man walks towards the window, the profile and activity identifier module 213 of the human wellness recommendation system 103 identifies a physical profile of the young man present in the room using UWB based sensors and historic physical profile stored in the database 113.

Thereafter, the profile and activity identifier module 213 monitors activity of the young man identified in the room using the UWB based sensors 101 and historic activity profile stored in the database 113. In this case, the profile and activity identifier module 213 identifies that the young man is walking towards the window in the room. The output 813 of the profile and activity identifier module 213 is sent to the device and event correlation module 215. The device and event correlation module 215 of the human wellness recommendation system 103 tracks operational state of one or more loT devices in the room. In this case, the device and event correlation module 215 tracks the operational state of the weather sensor 801 in the room. Using the output 813 of the profile and activity identifier module 213, an output 803 of the UWB based sensors 101 and the operational state of the weather sensor 801 in the room, the device and event correlation module 215 correlates the physical profile of the young man with at least one of the current activity of the young man, the operational state of the weather sensor, the output of the UWB based sensors 101, and room (i.e., associated location of the weather sensor). The wellness analyser 217 of the human wellness recommendation system 103 predicts a potential anomalous event 815 using the correlated output 807 from the device and event correlation module 215. In this case, using the surrounding awareness that window is open and adverse weather condition, the wellness analyser 217 predicts a potential anomalous event 815 (i.e., no possibility of health risk) of the young man in the room.

Thereafter, the alert generator 219 of the human wellness recommendation system 103 provides a wellness solution 817 to the young man based on the predicted potential anomalous event 815 and similar historic event identified in past at different location within the loT environment and corresponding action performed by the young man to the similar historic event. In this case, the alert generator 219 turns the speaker 115 to ON state to play the wellness solution 817 "Please close the window"

According to an embodiment, a method for providing human wellness recommendation based on Ultra-Wideband (UWB) based human activity detection, the method may include identifying a physical profile of each user present in an Internet of Things (IoT) environment. The method may include monitoring a current activity of each user in the loT environment and one or more locations associated with the current activity. The method may include tracking an operational state of one or more loT devices at the one or more locations within the loT environment. The method may include predicting a potential anomalous event by correlating the physical profile of each user with at least one of the current activity of each user, the one or more locations associated with the current activity, a state of environment at the one or more locations associated, and the operational state of the one or more loT devices. The method may include providing at least one of wellness risk alert and/or wellness solution to at least one user identified in the IoT environment based on the predicted potential anomalous event.

According to an embodiment, the physical profile of each user is identified using UWB based sensors(101) and a historic physical profile stored in a database(113).According to an embodiment, the identifying of the physical profile of each user present in the loT environment may be performed using multiple angular data(213-1) received from UWB based sensors(101) in a time-series manner and using a reinforcement learning technique involving feedback from a user of the loT environment.

According to an embodiment, the monitoring of the current activity of each user in the loT environment is performed using multiple angular data(213-1) received from UWB based sensors(101) in a time-series manner and using Recurrent Neural Network (RNN) technique based classification.

According to an embodiment the at least one activity includes a physical activity and is selected from a group comprising walking, moving, running, crawling, sitting, standing, jumping, bending, cleaning, or eating.

According to an embodiment, the physical profile and the historic physical profile both comprise information associated with at least one of user height, user body type, user shape, user age, user gender and stage type, user movement, user average speed of movement, or restricted user movement.

According to an embodiment, correlating the physical profile of each user with the at least one of the current activity of each user, the one or more locations associated with the current activity, the state of environment at the one or more locations associated, and the operational state of the one or more loT devices is performed using a supervised machine learning technique.

According to an embodiment, the providing of the at least one of wellness risk alert and/or wellness solution to the at least one user identified in the loT environment is based on the predicted potential anomalous event and similar historic event identified in the past at different location within the loT environment and corresponding action performed by the one or more users to the similar historic event.

According to an embodiment, the providing of the at least one of wellness risk alert and/or wellness solution to the at least one user identified in the loT environment is performed using a classification technique.

According to an embodiment, the at least one of wellness risk alert and/or wellness solution is indicated using the at least one IoT device at the one or more locations within the loT environment.

According to an embodiment, the method may include determining an action performed by the one or more users on the one or more loT devices at the one or more locations in the loT environment to the at least one of wellness risk alert and/or wellness solution at the one or more locations. The method may include retraining the correlation between the physical profile of each user with at least one of the current activity of each user, the one or more locations associated with the current activity, the state of environment at the one or more locations associated, and the operational state of the one or more loT devices.

According to an embodiment, An electronic device for providing human wellness recommendation based on Ultra-Wideband (UWB) based human activity detection, the electronic device(103) may include at least one processor(105) and at least one memory(107) communicatively coupled to the at least one processor(105), and configured to store processor-executable instructions, which on execution. The at least one processor (105) may configure to identify a physical profile of each user present in an Internet of Things (IoT) environment. The at least one processor (105) may configure to monitor a current activity of each user in the loT environment and one and more locations associated with the current activity. The at least one processor (105) may configure to track an operational state of one or more loT devices at the one or more locations within the loT environment. The at least one processor (105) may configure to predict a potential anomalous event by correlating the physical profile of each user with at least one of the current activity of each user, the one or more locations associated with the current activity, a state of environment at the one or more locations associated, and the operational state of the one or more IoT devices. The at least one processor (105) may configure to provide at least one of wellness risk alert and/or wellness solution to at least one user identified in the loT environment based on the predicted potential anomalous event.According to an embodiment, the at least one processor (105) may configure to identify the physical profile of each user using UWB based sensors(101) and a historic physical profile stored in a database(113).

According to an embodiment, the identifying of the physical profile of each user present in the loT environment is performed using multiple angular data (213-1) received from UWB based sensors(101) in a time-series manner and using a reinforcement learning technique involving feedback from a user of the loT environment.

According to an embodiment, a computer-readable storage medium, having a computer program stored thereon that performs, when executed by a processor. The medium may include a computer program to identify a physical profile of each user present in an Internet of Things (IoT) environment. The medium may include a computer program to monitor a current activity of each user in the loT environment and one and more locations associated with the current activity. The medium may include a computer program to track an operational state of one or more loT devices at the one or more locations within the loT environment. The medium may include predict a potential anomalous event by correlating the physical profile of each user with at least one of the current activity of each user, the one or more locations associated with the current activity, a state of environment at the one or more locations associated, and the operational state of the one or more loT devices. The medium may include provide at least one of wellness risk alert and/or wellness solution to at least one user identified in the loT environment based on the predicted potential anomalous event.

An embodiment of the disclosure may be to address at least the above-mentioned problems and/or disadvantages and to provide at least the advantages described below. Accordingly, an aspect of the disclosure is to provide a method and a human wellness recommendation system for providing human wellness recommendation using Ultra-Wideband (UWB) based human activity detection.

An embodiment of the disclosure may be set forth in part in the description which follows and, in part, will be apparent from the description, or may be learned by practice of the presented embodiments.

According to an embodiment, a method for providing human wellness recommendation using Ultra-Wideband (UWB) based human activity detection is provided. The method includes identifying, by an electronic device, a physical profile of each user present in an Internet of Things (IoT) environment, monitoring, by the electronic device, a current activity of each user identified in the loT environment and a location associated with the current activity, tracking, by the electronic device an operational state of one or more loT devices at one or more locations within the loT environment, predicting, by the electronic device, a potential anomalous event by correlating the physical profile of each user with at least one of the current activity of each user, a state of environment of the location associated with the current activity, and the operational state of the one or more loT devices, and providing, by the electronic device at least one wellness risk alert to at least one user identified in the loT environment based on the predicted potential anomalous event.

According to an embodiment, an electronic device for providing human wellness recommendation using Ultra-Wideband (UWB) based human activity detection is provided. The electronic device includes a processor, and a memory communicatively coupled to the processor, and configured to store processor-executable instructions, which on execution, cause the processor to identify a physical profile of each user present in an Internet of Things (IoT) environment, monitor a current activity of each user identified in the loT environment and a location associated with the current activity, track an operational state of one or more loT devices at one or more locations within the loT environment, to predict a potential anomalous event by correlating the physical profile of each user with at least one of the current activity of each user, a state of environment of the location associated with the current activity, and the operational state of the one or more loT devices, and to provide at least one wellness risk alert to at least one user identified in the loT environment based on the predicted potential anomalous event.

According to an embodiment, a system for providing human wellness recommendation using Ultra-Wideband (UWB) based human activity detection is provided. The system includes an electronic device as described above. The electronic device further includes a processor, and a memory communicatively coupled to the processor, wherein the memory stores processor-executable instructions, which on execution, cause the processor to identify a physical profile of each user present in an Internet of Things (IoT) environment. Further, the processor is configured to identify a current activity of each user identified in the loT environment and a location associated with the current activity. Subsequently, the processor is configured to track an operational state of one or more loT devices at one or more locations within the loT environment. Thereafter, the processor is configured to predict a potential anomalous event by correlating the physical profile of each user with at least one of the current activity of each user, a state of environment of the location associated with the current activity, and the operational state of the one or more loT devices. Lastly, the processor is configured to provide at least one wellness risk alert to at least one user identified in the loT environment based on the predicted potential anomalous event.

According to an embodiment, advantages, and salient features of the disclosure will become apparent to those skilled in the art from the following detailed description, which, taken in conjunction with the annexed drawings, discloses various embodiments of the disclosure.

According to an embodiment of the disclosure, the disclosure may relate to a system for providing human wellness recommendation based on Ultra-Wideband (UWB) based human activity detection. The system comprises an electronic device 103 as described above. The electronic device 103 further comprises a processor 105, and a memory 107 communicatively coupled to the processor 105, wherein the memory 107 stores processor-executable instructions, which on execution, cause the processor 105 to identify a physical profile of each user present in an Internet of Things (IoT) environment. Further, the processor 105 is configured to monitor a current activity of each user identified in the loT environment and a location associated with the current activity. Subsequently, the processor 105 is configured to track operational state of one or more loT devices at one or more locations within the loT environment. Thereafter, the processor 105 is configured to predict a potential anomalous event by correlating the physical profile of each user with at least one of the current activity of each user, a state of environment of the location associated with the current activity, and the operational state of the one or more loT devices. Lastly, the processor 105 is configured to provide at least one wellness risk alert to at least one user identified in the loT environment based on the predicted potential anomalous event.

Some advantages of the disclosure are given below:

An embodiment of the disclosure provides a human wellness recommendation system that allows user to be always identified anywhere in an IoT environment irrespective of surrounding (e.g., low light condition, user behind an object, under the table, corner, and the like) based on the unique physical profile learnt over time using UWB based sensors installed in the loT environment.

The UWB based sensors have centimeter grade accuracy and are relatively cheaper to install in multiple rooms as compared camera based or Light Detection and Ranging (LIDAR) based or Dynamic Vision Sensor (DVS) based user profile and movement tracking.

Since the disclosure uses physical profile to identify a user in the loT environment, there is no privacy issue and no dependency on facial recognition. Furthermore, use of the physical profile to identify a user is more accurate than the facial recognition as there are possibilities that face of a user may not be always recognizable due to poor image quality. Also, the disclosure does not require user to be wearing any sensor or wearable device to receive at least one of the wellness risk alert and/or the wellness solution.

Use of multiple angular data from UWB based sensors for identifying a physical profile of each user and for monitoring activity of each user requires less computation or processing as compared to image/video frame processing.

Use of multiple angular data such as SS, ToA, TDA and AoA of the UWB based sensors allows tracking of stationary and non-stationary object's size, position, speed and direction to predict collision or vulnerability path for better awareness of the loT environment. This approach is efficient for continuous tracking of users, even in corner location room or users obstructed by objects in front of them due to high penetration depth of the UWB based sensors. Furthermore, use of UWB based sensors have no limitation of angle of view.

An embodiment of the disclosure uses a historic event identified in the past at a different location within an IoT environment and corresponding action performed by one or more users to the similar historic event to provide at least one of a risk alert and/or a solution to the one or more users at a current location in the loT environment. Furthermore, based on the action performed by the one or more users to the at least one of the risk alert and/or the solution, the human wellness recommendation system is continuously retrained to reduce future unpleasant experience or event for the one or more users.

The described operations may be implemented as a method, system or article of manufacture using standard programming and/or engineering techniques to produce software, firmware, hardware, or any combination thereof. The described operations may be implemented as code maintained in a "non-transitory computer readable medium" where a processor may read and execute the code from the computer readable medium. The processor is at least one of a microprocessor and a processor capable of processing and executing the queries. A non-transitory computer readable medium may include media such as magnetic storage medium (e.g., hard disk drives, floppy disks, tape, etc.), optical storage (CD-ROMs, DVDs, optical disks, etc.), volatile and non-volatile memory devices (e.g., EEPROMs, ROMs, PROMs, RAMs, DRAMs, SRAMs, Flash Memory, firmware, programmable logic, etc.), etc. Further, non-transitory computer-readable media include all computer-readable media except for a transitory. The code implementing the described operations may further be implemented in hardware logic (e.g., an integrated circuit chip, Programmable Gate Array (PGA), Application Specific Integrated Circuit (ASIC), etc.).

The terms "an embodiment", "embodiment", "embodiments", "the embodiment", "the embodiments", "one or more embodiments", "some embodiments", and "one embodiment" mean "one or more (but not all) embodiments of the disclosures" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

A description of an embodiment with several components in communication with each other does not imply that all such components are required. On the contrary, a variety of optional components are described to illustrate the wide variety of possible embodiments of the disclosure.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features. Thus, other embodiments of the disclosure need not include the device itself.

The illustrated operations of FIG. 3 show certain events occurring in a certain order. In alternative embodiments, certain operations may be performed in a different order, modified or removed. Moreover, steps may be added to the above-described logic and still conform to the described embodiments. Further, operations described herein may occur sequentially or certain operations may be processed in parallel. Yet further, operations may be performed by a single processing unit or by distributed processing units.

Finally, the language used in the specification has been principally selected for readability and instructional purposes, and it may not have been selected to delineate or circumscribe the inventive subject matter. It is therefore intended that the scope of the disclosure be limited not by this detailed description, but rather by any claims that issue on an application based here on. Accordingly, the disclosure of the embodiments of the disclosure is intended to be illustrative, but not limiting, of the scope of the disclosure, which is set forth in the following claims.

While the disclosure has been shown and described with reference to various embodiments thereof, it will be understood by those skilled in the art that various changes in form and details may be made therein without departing from the scope of the disclosure as defined by the appended claims.

## Claims

1. A method performed by an electronic device for providing human wellness recommendations based on Ultra-Wideband, UWB, based human activity detection, the method comprising:
identifying (301) a physical profile of each user present in an Internet of Things, IoT, environment;
monitoring (303) a current activity of each user in the loT environment and one or more locations associated with the current activity;
tracking (305) an operational state of one or more loT devices (115) at the one or more locations within the loT environment;
predicting (307) a potential anomalous event by correlating the identified physical profile of each user with the current activity of each user, the one or more locations associated with the current activity, a state of environment at the one or more locations associated with the current activity, and the operational state of the one or more loT devices; and
providing (309) at least one of a risk alert and a message to at least one user identified in the loT environment based on the predicted potential anomalous event and a similar historic event identified in the past at a different location within the loT environment and corresponding action performed by the one or more users to the similar historic event,
wherein the risk alert is an alert indicated through a sound or through light, and the message is a solution outputted through a speaker of the electronic device.

2. The method of claim 1, wherein the physical profile of each user is identified using UWB based sensors (101) and a historic physical profile stored in a database (113).

3. The method of any one of the preceding claims, wherein the identifying of the physical profile of each user present in the loT environment is performed using multiple angular data (213-1) received from UWB based sensors (101) in a time-series manner and using a reinforcement learning technique involving feedback from a user of the loT environment.

4. The method of any one of the preceding claims, wherein the monitoring of the current activity of each user in the loT environment is performed using multiple angular data (213-1) received from UWB based sensors (101) in a time-series manner and using Recurrent Neural Network, RNN, technique based classification.

5. The method of any one of the preceding claims, wherein the current activity includes a physical activity and is selected from a group comprising walking, moving, running, crawling, sitting, standing, jumping, bending, cleaning, or eating.

6. The method of claim 2, wherein the physical profile and the historic physical profile both comprise information associated with at least one of user height, user body type, user shape, user age, user gender and stage type, user movement, user average speed of movement, or restricted user movement.

7. The method of any one of the preceding claims, wherein correlating the physical profile of each user with the at least one of the current activity of each user, the one or more locations associated with the current activity, the state of environment at the one or more locations associated, and the operational state of the one or more loT devices is performed using a supervised machine learning technique.

8. The method of any one of the preceding claims, wherein the providing of the at least one of the risk alert and the message to the at least one user identified in the loT environment is performed using a classification technique.

9. The method of any one of the preceding claims, wherein the at least one of the risk alert and the message is indicated using the at least one loT device present at the one or more locations within the loT environment.

10. The method of any one of the preceding claims, further comprising:
determining an action performed by the one or more users on the one or more loT devices at the one or more locations in the loT environment to the at least one of the risk alert and the message at the one or more locations; and
retraining the correlation between the physical profile of each user with at least one of the current activity of each user, the one or more locations associated with the current activity, the state of environment at the one or more locations associated, and the operational state of the one or more loT devices.

11. An electronic device for providing human wellness recommendations based on Ultra-Wideband, UWB, based human activity detection, the electronic device(103) comprising:
at least one processor (105); and
at least one memory (107) communicatively coupled to the at least one processor (105), and configured to store processor-executable instructions, which on execution, cause the at least one processor (105) to:
identify a physical profile of each user present in an Internet of Things, loT, environment,
monitor a current activity of each user in the loT environment and one and more locations associated with the current activity,
track an operational state of one or more loT devices (115) at the one or more locations within the loT environment,
predict a potential anomalous event by correlating the identified physical profile of each user with the current activity of each user, the one or more locations associated with the current activity, a state of environment at the one or more locations associated with the current activity, and the operational state of the one or more loT devices; and
provide at least one of a risk alert and a message to at least one user identified in the loT environment based on the predicted potential anomalous event and a similar historic event identified in the past at a different location within the loT environment and corresponding action performed by the one or more users to the similar historic event,
wherein the risk alert is an alert indicated through a sound or through light and the message is a solution outputted through a speaker of the electronic device.

12. The electronic device (101) of any one of the preceding claims, wherein the instructions further include instructions to identify the physical profile of each user using UWB based sensors(101) and a historic physical profile stored in a database (113).

13. The electronic device (101) of any one of the preceding claims, wherein the identifying of the physical profile of each user present in the loT environment is performed using multiple angular data (213-1) received from UWB based sensors (101) in a time-series manner and using a reinforcement learning technique involving feedback from a user of the loT environment.

14. A computer-readable storage medium, having a computer program stored thereon that performs, when executed by a processor, the method according to any one of claims 1 to 10.

## Patentansprüche

1. Von einer elektronischen Vorrichtung durchgeführtes Verfahren zur Bereitstellung von Empfehlungen für menschliche Wohlbefinden auf Basis von Ultrabreitband-, UWB, -basierter Erkennung menschlicher Aktivitäten, wobei das Verfahren Folgendes umfasst:
Identifizieren (301) eines physischen Profils jedes in einer Internet-of-Things-, IoT, - Umgebung anwesenden Benutzers;
Überwachen (303) einer aktuellen Aktivität jedes Benutzers in der IoT-Umgebung und eines oder mehrerer Standorte, die mit der aktuellen Aktivität assoziiert sind;
Verfolgen (305) eines Betriebszustands eines oder mehrerer IoT-Vorrichtungen (115) an dem einen oder den mehreren Standorten innerhalb der IoT-Umgebung;
Vorhersagen (307) eines potenziellen anomalen Ereignisses durch Korrelieren des identifizierten physischen Profils jedes Benutzers mit der aktuellen Aktivität jedes Benutzers, dem einen oder den mehreren mit der aktuellen Aktivität assoziierten Standorten, einem Zustand der Umgebung an dem einen oder den mehreren mit der aktuellen Aktivität assoziierten Standorten und dem Betriebszustand der einen oder der mehreren IoT-Vorrichtungen; und
Bereitstellen (309) mindestens einer von einer Risikowarnung und einer Nachricht an mindestens einen in der IoT-Umgebung identifizierten Benutzer auf der Grundlage des vorhergesagten potenziellen anomalen Ereignisses und eines ähnlichen historischen Ereignisses, das in der Vergangenheit an einem anderen Standort innerhalb der IoT-Umgebung identifiziert wurde, sowie der entsprechenden Aktion, die von dem einen oder den mehreren Benutzern gegenüber dem ähnlichen historischen Ereignis durchgeführt wurde,
wobei die Risikowarnung eine durch einen Ton oder durch Licht angegebene Warnung und die Nachricht eine über einen Lautsprecher der elektronischen Vorrichtung ausgegebene Lösung ist.

2. Verfahren nach Anspruch 1, wobei das physische Profil jedes Benutzers unter Verwendung von UWB-basierten Sensoren (101) und eines in einer Datenbank (113) gespeicherten historischen physischen Profils identifiziert wird.

3. Verfahren nach einem der vorstehenden Ansprüche, wobei die Identifizierung des physischen Profils jedes in der IoT-Umgebung anwesenden Benutzers unter Verwendung mehrerer Winkeldaten (213-1), die zeitreihenweise von UWB-basierten Sensoren (101) empfangen werden, sowie unter Verwendung einer Technik des verstärkenden Lernens, die Rückmeldungen von einem Benutzer der IoT-Umgebung einbezieht, durchgeführt wird.

4. Verfahren nach einem der vorstehenden Ansprüche, wobei die Überwachung der aktuellen Aktivität jedes Benutzers in der IoT-Umgebung unter Verwendung mehrerer Winkeldaten (213-1), die zeitreihenweise von UWB-basierten Sensoren (101) empfangen werden, sowie unter Verwendung einer auf der Recurrent Neural Network-, RNN, -Technik basierenden Klassifizierung durchgeführt wird.

5. Verfahren nach einem der vorstehenden Ansprüche, wobei die aktuelle Aktivität eine physische Aktivität umfasst und aus einer Gruppe ausgewählt wird, die Gehen, Bewegen, Laufen, Krabbeln, Sitzen, Stehen, Springen, Beugen, Putzen oder Essen umfasst.

6. Verfahren nach Anspruch 2, wobei das physische Profil und das historische physische Profil beide Informationen umfassen, die mit mindestens einem von Folgendem assoziiert sind: Körpergröße des Benutzers, Körperbau des Benutzers, Körperform des Benutzers, Alter des Benutzers, Geschlecht des Benutzers und Art der Bewegungsphase, Bewegung des Benutzers, durchschnittliche Bewegungsgeschwindigkeit des Benutzers und eingeschränkte Bewegung des Benutzers.

7. Verfahren nach einem der vorstehenden Ansprüche, wobei die Korrelation des physischen Profils jedes Benutzers mit mindestens einem von Folgendem - der aktuellen Aktivität jedes Benutzers, dem einen oder den mehreren mit der aktuellen Aktivität assoziierten Standorten, dem Zustand der Umgebung an dem einen oder den mehreren assoziierten Standorten und dem Betriebszustand der einen oder der mehreren IoT-Vorrichtungen - unter Verwendung einer überwachten maschinellen Lerntechnik durchgeführt wird.

8. Verfahren nach einem der vorstehenden Ansprüche, wobei die Bereitstellung der mindestens einen von der Risikowarnung und der Nachricht an den mindestens einen in der IoT-Umgebung identifizierten Benutzer unter Verwendung einer Klassifizierungstechnik durchgeführt wird.

9. Verfahren nach einem der vorstehenden Ansprüche, wobei die mindestens eine von der Risikowarnung und der Nachricht unter Verwendung der mindestens einen an dem einen oder den mehreren Standorten innerhalb der IoT-Umgebung vorhandenen IoT-Vorrichtung angegeben wird.

10. Verfahren nach einem der vorstehenden Ansprüche, umfassend ferner:
Bestimmen einer Aktion, die von dem einen oder den mehreren Benutzern auf der einen oder den mehreren IoT-Vorrichtungen an dem einen oder den mehreren Standorten in der IoT-Umgebung als Reaktion auf die mindestens eine von der Risikowarnung und der Nachricht an dem einen oder den mehreren Standorten durchgeführt wird; und
Neutrainieren der Korrelation zwischen dem physischen Profil jedes Benutzers mit mindestens einem von Folgendem: der aktuellen Aktivität jedes Benutzers, dem einen oder den mehreren Standorten, die mit der aktuellen Aktivität assoziiert sind, dem Zustand der Umgebung an dem einen oder den mehreren assoziierten Standorten sowie dem Betriebszustand der einen oder der mehreren IoT-Vorrichtungen.

11. Elektronische Vorrichtung zur Bereitstellung von Empfehlungen für menschliche Wohlbefinden auf Basis von Ultrabreitband-, UWB, -basierter Erkennung menschlicher Aktivitäten, wobei die elektronische Vorrichtung (103) Folgendes umfasst:
mindestens einen Prozessor (105); und
mindestens einen Speicher (107), der kommunikativ mit dem mindestens einen Prozessor (105) gekoppelt und dazu konfiguriert ist, vom Prozessor ausführbare Befehle zu speichern, die bei ihrer Ausführung den mindestens einen Prozessor (105) dazu veranlassen:
ein physisches Profil jedes in einer Internet-of-Things-, IoT, -Umgebung anwesenden Benutzers zu identifizieren,
eine aktuelle Aktivität jedes Benutzers in der IoT-Umgebung sowie einen oder mehrere mit der aktuellen Aktivität assoziierte Standorte zu überwachen,
einen Betriebszustand einer oder mehrerer IoT-Vorrichtungen (115) an dem einen oder den mehreren Standorten innerhalb der IoT-Umgebung zu verfolgen,
ein potenzielles anomales Ereignis durch Korrelieren des identifizierten physischen Profils jedes Benutzers mit der aktuellen Aktivität jedes Benutzers, dem einen oder den mehreren mit der aktuellen Aktivität assoziierten Standorten, einem Zustand der Umgebung an dem einen oder den mehreren mit der aktuellen Aktivität assoziierten Standorten und dem Betriebszustand der einen oder der mehreren IoT-Vorrichtungen vorherzusagen; und
mindestens eine von einer Risikowarnung und einer Nachricht an mindestens einen in der IoT-Umgebung identifizierten Benutzer auf der Grundlage des vorhergesagten potenziellen anomalen Ereignisses und eines ähnlichen historischen Ereignisses, das in der Vergangenheit an einem anderen Standort innerhalb der IoT-Umgebung identifiziert wurde, sowie der entsprechenden Aktion, die von dem einen oder den mehreren Benutzern gegenüber dem ähnlichen historischen Ereignis durchgeführt wurde, bereitzustellen,
wobei die Risikowarnung eine durch einen Ton oder durch Licht angegebene Warnung und die Nachricht eine über einen Lautsprecher der elektronischen Vorrichtung ausgegebene Lösung ist.

12. Elektronische Vorrichtung (101) nach einem der vorstehenden Ansprüche, wobei die Befehle ferner Befehle zum Identifizieren des physischen Profils jedes Benutzers unter Verwendung von UWB-basierten Sensoren (101) und eines in einer Datenbank (113) gespeicherten historischen physischen Profils umfassen.

13. Elektronische Vorrichtung (101) nach einem der vorstehenden Ansprüche, wobei die Identifizierung des physischen Profils jedes in der IoT-Umgebung anwesenden Benutzers unter Verwendung mehrerer Winkeldaten (213-1), die zeitreihenweise von UWB-basierten Sensoren (101) empfangen werden, sowie unter Verwendung einer Technik des verstärkenden Lernens, die Rückmeldungen von einem Benutzer der IoT-Umgebung einbezieht, durchgeführt wird.

14. Computerlesbares Speichermedium, auf dem ein Computerprogramm gespeichert ist, das bei Ausführung durch einen Prozessor das Verfahren nach einem der Ansprüche 1 bis 10 ausführt.

## Revendications

1. Procédé mis en œuvre par un dispositif électronique pour fournir une recommandation en matière de bien-être humain sur la base d'une détection d'activité humaine basée sur une bande ultra large, UWB, le procédé comprenant :
l'identification (301) d'un profil physique de chaque utilisateur présent dans un environnement de l'Internet des objets, IoT ;
la surveillance (303) d'une activité actuelle de chaque utilisateur dans l'environnement IoT et d'un ou plusieurs emplacements associés à l'activité actuelle ;
le suivi (305) d'un état de fonctionnement d'un ou plusieurs dispositifs IoT (115) au niveau des un ou plusieurs emplacements dans l'environnement IoT ;
la prédiction (307) d'un événement anormal potentiel par mise en corrélation du profil physique identifié de chaque utilisateur avec l'activité actuelle de chaque utilisateur, les un ou plusieurs emplacements associés à l'activité actuelle, un état de l'environnement au niveau des un ou plusieurs emplacements associés à l'activité actuelle, et l'état de fonctionnement des un ou plusieurs dispositifs IoT ; et
la fourniture (309) d'au moins l'une parmi une alerte de risque et un message à au moins un utilisateur identifié dans l'environnement IoT sur la base de l'événement anormal potentiel prédit et d'un événement historique similaire identifié dans le passé à un emplacement différent dans l'environnement IoT et d'une action correspondante effectuée par les un ou plusieurs utilisateurs en réponse à l'événement historique similaire,
dans lequel l'alerte de risque est une alerte indiquée par un son ou par une lumière, et le message est une solution émise par l'intermédiaire d'un haut-parleur du dispositif électronique.

2. Procédé selon la revendication 1, dans lequel le profil physique de chaque utilisateur est identifié à l'aide de capteurs basés sur l'UWB (101) et d'un profil physique historique stocké dans une base de données (113).

3. Procédé selon l'une quelconque des revendications précédentes, dans lequel l'identification du profil physique de chaque utilisateur présent dans l'environnement IoT est effectuée à l'aide de multiples données angulaires (213-1) reçues de capteurs basés sur l'UWB (101) de manière chronologique et à l'aide d'une technique d'apprentissage par renforcement impliquant une rétroaction provenant d'un utilisateur de l'environnement IoT.

4. Procédé selon l'une quelconque des revendications précédentes, dans lequel la surveillance de l'activité actuelle de chaque utilisateur dans l'environnement IoT est effectuée à l'aide de multiples données angulaires (213-1) reçues de capteurs basés sur l'UWB (101) de manière chronologique et à l'aide d'une classification basée sur une technique de réseau neuronal récurrent, RNN.

5. Procédé selon l'une quelconque des revendications précédentes, dans lequel l'activité actuelle comprend une activité physique et est sélectionnée parmi un groupe comprenant la marche, le déplacement, la course, le déplacement à quatre pattes, la position assise, la position debout, le saut, la flexion, le nettoyage ou l'alimentation.

6. Procédé selon la revendication 2, dans lequel le profil physique et le profil physique historique comprennent tous deux des informations associées à au moins l'un parmi la taille de l'utilisateur, le type corporel de l'utilisateur, la forme de l'utilisateur, l'âge de l'utilisateur, le sexe de l'utilisateur et le type de stade, le mouvement de l'utilisateur, la vitesse moyenne de déplacement de l'utilisateur ou le mouvement restreint de l'utilisateur.

7. Procédé selon l'une quelconque des revendications précédentes, dans lequel la mise en corrélation du profil physique de chaque utilisateur avec au moins l'un parmi l'activité actuelle de chaque utilisateur, les un ou plusieurs emplacements associés à l'activité actuelle, l'état de l'environnement au niveau des un ou plusieurs emplacements associés, et l'état de fonctionnement des un ou plusieurs dispositifs IoT est effectuée à l'aide d'une technique d'apprentissage automatique supervisé.

8. Procédé selon l'une quelconque des revendications précédentes, dans lequel la fourniture d'au moins l'une parmi l'alerte de risque et le message à l'au moins un utilisateur identifié dans l'environnement IoT est effectuée à l'aide d'une technique de classification.

9. Procédé selon l'une quelconque des revendications précédentes, dans lequel au moins l'une parmi l'alerte de risque et le message est indiquée à l'aide de l'au moins un dispositif IoT présent au niveau des un ou plusieurs emplacements dans l'environnement IoT.

10. Procédé selon l'une quelconque des revendications précédentes, comprenant en outre :
la détermination d'une action effectuée par les un ou plusieurs utilisateurs sur les un ou plusieurs dispositifs IoT au niveau des un ou plusieurs emplacements dans l'environnement IoT en réponse à au moins l'une parmi l'alerte de risque et le message au niveau des un ou plusieurs emplacements ; et
le réentraînement de la corrélation entre le profil physique de chaque utilisateur et au moins l'un parmi l'activité actuelle de chaque utilisateur, les un ou plusieurs emplacements associés à l'activité actuelle, l'état de l'environnement au niveau des un ou plusieurs emplacements associés, et l'état de fonctionnement des un ou plusieurs dispositifs IoT.

11. Dispositif électronique pour fournir une recommandation en matière de bien-être humain sur la base d'une détection d'activité humaine basée sur une bande ultra large, UWB, le dispositif électronique (103) comprenant :
au moins un processeur (105) ; et
au moins une mémoire (107) couplée de manière communicante à l'au moins un processeur (105), et configurée pour stocker des instructions exécutables par processeur, qui, lors de leur exécution, amènent l'au moins un processeur (105) à :
identifier un profil physique de chaque utilisateur présent dans un environnement de l'Internet des objets, IoT,
surveiller une activité actuelle de chaque utilisateur dans l'environnement IoT et un ou plusieurs emplacements associés à l'activité actuelle,
suivre un état de fonctionnement d'un ou plusieurs dispositifs IoT (115) au niveau des un ou plusieurs emplacements dans l'environnement IoT,
prédire un événement anormal potentiel par mise en corrélation du profil physique identifié de chaque utilisateur avec l'activité actuelle de chaque utilisateur, les un ou plusieurs emplacements associés à l'activité actuelle, un état de l'environnement au niveau des un ou plusieurs emplacements associés à l'activité actuelle, et l'état de fonctionnement des un ou plusieurs dispositifs IoT ; et
fournir au moins l'une parmi une alerte de risque et un message à au moins un utilisateur identifié dans l'environnement IoT sur la base de l'événement anormal potentiel prédit et d'un événement historique similaire identifié dans le passé à un emplacement différent dans l'environnement IoT et d'une action correspondante effectuée par les un ou plusieurs utilisateurs en réponse à l'événement historique similaire,
dans lequel l'alerte de risque est une alerte indiquée par un son ou par une lumière et le message est une solution émise par l'intermédiaire d'un haut-parleur du dispositif électronique.

12. Dispositif électronique (101) selon l'une quelconque des revendications précédentes, dans lequel les instructions comprennent en outre des instructions pour identifier le profil physique de chaque utilisateur à l'aide de capteurs basés sur l'UWB (101) et d'un profil physique historique stocké dans une base de données (113).

13. Dispositif électronique (101) selon l'une quelconque des revendications précédentes, dans lequel l'identification du profil physique de chaque utilisateur présent dans l'environnement IoT est effectuée à l'aide de multiples données angulaires (213-1) reçues de capteurs basés sur l'UWB (101) de manière chronologique et à l'aide d'une technique d'apprentissage par renforcement impliquant une rétroaction provenant d'un utilisateur de l'environnement IoT.

14. Support de stockage lisible par ordinateur, ayant un programme informatique stocké sur celui-ci qui met en œuvre, lorsqu'il est exécuté par un processeur, le procédé selon l'une quelconque des revendications 1 à 10.
